# EUROPEAN PATENT SPECIFICATION

(11) **EP 3 929 193 B1**
(45) Date of publication and mention of the grant of the patent: **23.10.2024**
(21) Application number: 20759116.5
(22) Date of filing: 21.02.2020
(51) Int. Cl.: C07D 405/14, C07D 209/82, C07D 263/62, C07D 277/66, C07D 251/24, H10K 50/844, H10K 85/60, H10K 50/858, H10K 85/30, C09K 11/06, H10K 50/11, H10K 101/10

(54) **ORGANIC ELECTROLUMINESCENT ELEMENT COMPRISING A CAPPING LAYER WITH AN ARYLAMINE COMPOUND HAVING BENZOAZOLE RING STRUCTURE**
ORGANISCHES ELEKTROLUMINESZENTES ELEMENT MIT EINER DECKSCHICHT ENTHALTEND EINE ARYLAMINVERBINDUNG MIT BENZOAZOLRINGSTRUKTUR
ÉLÉMENT ÉLECTROLUMINESCENT ORGANIQUE AVEC UNE COUCHE DE RECOUVREMENT CONTENANT UN COMPOSÉ ARYLAMINE AYANT UNE STRUCTURE CYCLIQUE DE BENZOAZOLE

(30) Priority: 22.02.2019 JP 2019030036
(43) Date of publication of application: 29.12.2021
(73) Proprietor: Hodogaya Chemical Co., Ltd., Tokyo 104-0028 (JP); SFC Co., Ltd., Cheongju-si, Chungcheongbuk-do 28122 (KR)
(72) Inventor: MOCHIZUKI, Shunji, Tokyo 104-0028 (JP); UEHARA, Takuya, Tokyo 104-0028 (JP); KASE, Kouki, Tokyo 104-0028 (JP); HIRAYAMA, Yuta, Tokyo 104-0028 (JP); YAMAMOTO, Takeshi, Tokyo 104-0028 (JP); HAYASHI, Shuichi, Tokyo 104-0028 (JP); CHOI, Young-Tae, Cheongju-si, Chungcheongbuk-do 28122 (KR); LEE, Se-Jin, Cheongju-si, Chungcheongbuk-do 28122 (KR); PARK, Seok-Bae, Cheongju-si, Chungcheongbuk-do 28122 (KR); YU, Tae-Jung, Cheongju-si, Chungcheongbuk-do 28122 (KR); YANG, Byung-Sun, Cheongju-si, Chungcheongbuk-do 28122 (KR)
(74) Representative: Forstmeyer, Dietmar
(86) International application number: PCT/JP2020/007158
(87) International publication number: WO 2020/171221

(56) References cited:
- EP-A1- 3 432 688
- EP-A1- 3 904 487
- CN-A- 107 868 067
- CN-A- 107 868 067
- CN-A- 107 973 786
- JP-A- 2008 069 120
- JP-A- 2013 028 597
- JP-A- 2015 092 485
- JP-A- 2019 500 314
- KR-A- 20190 007 257
- US-A1- 2015 200 373
- US-A1- 2015 200 373
- US-A1- 2018 040 829

## Description

### Technical Field

The present invention relates to a compound suitable for an organic electroluminescence device (abbreviated hereinbelow as "organic EL device") which is a self-luminous light-emitting device suitable for various display devices, and to the organic EL device. More specifically, the present invention relates to an arylamine compound having a benzazole ring structure, and to an organic EL device employing the compound.

### Background Art

Organic EL devices, which are self-luminous light-emitting devices, are brighter, have better visibility, and are capable of clearer display compared to liquid crystal devices. Therefore, organic EL devices have been actively researched.

In 1987, C. W. Tang et al. of Eastman Kodak Company developed a device having a multilayer structure wherein various roles for light emission were allotted respectively to different materials, thereby achieving practical utilization of organic EL devices using organic materials. They developed a laminate including a layer of a fluorescent substance, tris(8-hydroxyquinoline)aluminum (abbreviated hereinbelow as "Alq3"), capable of transporting electrons and a layer of an aromatic amine compound capable of transporting holes. Injecting both charges into the fluorescent substance layer causes emission of light, achieving a high luminance of 1,000 cd/m² or higher at a voltage of 10 V or less (see, for example, Patent Literatures 1 and 2).

Many improvements have been heretofore made for practical utilization of organic EL devices. Further subdivision of the various roles within the multilayer structure has yielded an electroluminescent device wherein an anode, a hole injection layer, a hole transport layer, a light-emitting layer, an electron transport layer, an electron injection layer, and a cathode are formed sequentially on a substrate. Further, it has been found that bottom-emission light-emitting devices, which allow light to exit from the bottom side of such electroluminescent devices, can achieve high efficiency and high durability (see, for example, Non-Patent Literature 1).

Recent years have seen the use of top-emission light-emitting devices which allow light emission from the top side by using, as an anode, a metal having a high work function. In the aforementioned bottom-emission structure, light exits from the bottom side where pixel circuits are provided; this configuration unfortunately limits the area from which light can be extracted. In contrast, in top-emission light-emitting devices, light exits from the top side; this is advantageous in that the area for extracting light can be increased, since the pixel circuits do not get in the way.

To further improve luminous efficiency, attempts have been made to employ triplet excitons. Also, the use of phosphorescent substances has been investigated (see, for example, Non-Patent Literature 2).

The light-emitting layer can be prepared by doping a charge-transporting compound, typically called a "host material", with a fluorescent substance or a phosphorescent substance. In recent years, it has been found that the combined use, as a host, of a compound having a nitrogen-containing heteroaromatic ring structure and having high electron transportability and a compound having a carbazole structure and having hole transportability can significantly improve luminous efficiency compared to cases where they are used singly (see, for example, Patent Literatures 3 and 4).

Iridium complexes are typically used as materials exhibiting high luminous efficiency as phosphorescent substances. Unfortunately, the emission spectra of organic EL devices employing iridium complexes have broad half-widths, thus resulting in poor color purity.

As a means for improving color purity as well as luminous efficiency, a light-emitting device configuration has been proposed, wherein a "capping layer" having a high refractive index is provided on the outer side of a semi-transparent electrode having a low refractive index, to thereby constitute an optical resonator called a "microcavity" to adjust the emission spectrum (see, for example, Patent Literature 5).

Alq3 and arylamine compounds have been proposed as materials for the capping layer (see, for example, Non-Patent Literature 3 and Patent Literature 6). Unfortunately, these conventional materials have low refractive indices in the green and red emission ranges, thus resulting in poor light extraction efficiency. Patent Literature documents 13 and 14 also disclose organic EL devices having an organic capping layer.

Also, devices provided with a capping layer using such conventional materials allow transmission of sunlight within the wavelength range of 400 nm to 410 nm, which may affect materials inside the device. This may also impair color purity as well as light extraction efficiency.

Thus, in order to improve the characteristics of organic EL devices, and particularly to absorb sunlight within the wavelength range of 400 nm to 410 nm to prevent impact on materials inside the device, and also to significantly improve light extraction efficiency, there is a demand for a material, usable as a capping layer material, having a high refractive index and having excellent stability and durability in a thin film.

### Citation List

### Patent Literature

Patent Literature 1: US5792557
Patent Literature 2: US5639914
Patent Literature 3: EP3042943
Patent Literature 4: US20170104163
Patent Literature 5: US7102282
Patent Literature 6: US20140225100
Patent Literature 7: WO2015/001726
Patent Literature 8: US 10147891
Patent Literature 9: JP2002-105055A
Patent Literature 10: EP2730583
Patent Literature 11: US20180093962
Patent Literature 12: EP2684932
Patent Literature 13: EP3432688
Patent Literature 14: US2018040829

### Non-Patent Literature

Non-Patent Literature 1: Japan Society of Applied Physics, 9th Class, Proceedings, pp. 55-61 (2001)
Non-Patent Literature 2: Japan Society of Applied Physics, 9th Class, Proceedings, pp. 23-31 (2001)
Non-Patent Literature 3: Appl. Phys. Let., 78, 544 (2001)
Non-Patent Literature 4: J. Org. Chcm., 60, 7508 (1995)
Non-Patent Literature 5: Synth. Commun., 11, 513 (1981)

### Summary of Invention

An objective of the present invention is to provide an organic EL device that employs, in combination, various materials for an organic EL device that have excellent injection-transport performance of holes and electrons and have excellent stability and durability in a thin-film state, and a capping layer composed from a material that has a high light absorption coefficient to absorb sunlight within the wavelength range of 400 nm to 410 nm and thereby prevent impact on materials inside the organic EL device, has a high refractive index to significantly improve light extraction efficiency, has excellent stability, durability and light resistance in a thin film, and has no absorption within the wavelength ranges of blue, green and red, to thereby effectively achieve the characteristics of the respective materials in order to provide an organic EL device having (1) high luminous efficiency and high power efficiency, (2) a low emission start voltage, (3) a low practical driving voltage, and (4) a particularly long lifetime.

Physical characteristics of a capping layer material suitable for achieving the aforementioned objective may include, for example, (1) capability of absorbing light within the range from 400 nm to 410 nm, (2) a high refractive index, (3) capability of vapor deposition without thermal decomposition, (4) stability in a thin-film state, and (5) a high glass transition temperature.

Physical characteristics of a device suitable for the present invention may include, for example, (1) high light extraction efficiency, (2) no deterioration of color purity, (3) transmission of light with no change over time, (4) high luminous efficiency and power efficiency, (5) a low emission start voltage, (6) a low practical driving voltage, and (7) a particularly long lifetime.

To achieve the aforementioned objective, Inventors focused on the fact that arylamine materials have excellent stability and durability in a thin film, and that metal complexes, as phosphorescent dopants, have excellent luminous efficiency. Inventors produced various organic EL devices by selecting and using, as capping layer materials, specific benzazole-ring-structure arylamine compounds that have a high refractive index within the wavelength range of 400 nm to 650 nm and have a high extinction coefficient within the wavelength range of 400 nm to 410 nm, and by combining those compounds with phosphorescent dopant-containing light-emitting layers. Further, Inventors diligently assessed the characteristics of those devices, to thus accomplish the present invention.

An organic EL device of the present disclosure capable of solving the aforementioned problems includes at least an anode, a first hole transport layer, a second hole transport layer, a light-emitting layer, an electron transport layer, a cathode, and a capping layer in this order, wherein: the capping layer contains an arylamine compound having a group (benzazole ring structure) represented by the following formula (1); and the light-emitting layer contains the host compounds (A-43) and (B-25) and the phosphorescent dopant compound (4-35). 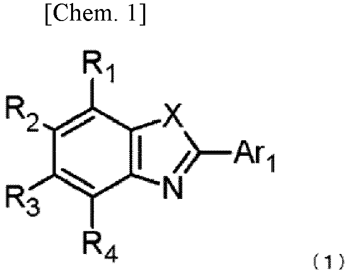
(In the formula, R₁ to R₄ each independently represent a hydrogen atom, a deuterium atom, a fluorine atom, a chlorine atom, a cyano group, a nitro group, a silyl group, a linear or branched alkyl group having 1 to 6 carbon atoms and optionally having a substituent, a cycloalkyl group having 5 to 10 carbon atoms and optionally having a substituent, a linear or branched alkenyl group having 2 to 6 carbon atoms and optionally having a substituent, a linear or branched alkyloxy group having 1 to 6 carbon atoms and optionally having a substituent, a cycloalkyloxy group having 5 to 10 carbon atoms and optionally having a substituent, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group;
Ar₁ represents a substituted or unsubstituted aromatic hydrocarbon group, a substituted or unsubstituted aromatic heterocyclic group, or a substituted or unsubstituted fused polycyclic aromatic group;
one of R₁ to R₄ and Ar₁ is a linking group as a bonding site;
X represents an oxygen atom or a sulfur atom; and
R₁ to R₄ may be bonded to each other via a single bond, a substituted or unsubstituted methylene group, a substituted or unsubstituted amino group, an oxygen atom, or a sulfur atom, to form a ring.)

The organic EL device of the present invention includes a capping layer provided on the outer side of a transparent or semi-transparent electrode and having a higher refractive index than the electrode, and is thus significantly improved in color purity and light extraction efficiency. Further, by using, for the capping layer, an arylamine compound having a group represented by formula (1), sunlight within the range of 400 to 410 nm can be absorbed, and thus, damage to the light-emitting device can be prevented. With these characteristics, the organic EL device can be suitably applied to full-color displays and can display images with high definition.

### Brief Description of Drawings

[Fig. 1] Fig. 1 is a diagram illustrating structures of compounds (2-1) to (2-16) as arylamine compounds represented by formula (2).
[Fig. 2] Fig. 2 is a diagram illustrating structures of compounds (2-17) to (2-32) as arylamine compounds represented by formula (2).
[Fig. 3] Fig. 3 is a diagram illustrating structures of compounds (2-33) to (2-48) as arylamine compounds represented by formula (2).
[Fig. 4] Fig. 4 is a diagram illustrating structures of compounds (2-49) to (2-64) as arylamine compounds represented by formula (2).
[Fig. 5] Fig. 5 is a diagram illustrating structures of compounds (2-65) to (2-82) as arylamine compounds represented by formula (2).
[Fig. 6] Fig. 6 is a diagram illustrating structures of compounds (3-1) to (3-14) as arylamine compounds represented by formula (3).
[Fig. 7] Fig. 7 is a diagram illustrating structures of compounds (3-15) to (3-30) as arylamine compounds represented by formula (3).
[Fig. 8] Fig. 8 is a diagram illustrating structures of compounds (3-31) to (3-42) as arylamine compounds represented by formula (3).
[Fig. 9] Fig. 9 is a diagram illustrating structures of compounds (3-43) to (3-53) as arylamine compounds represented by formula (3).
[Fig. 10] Fig. 10 is a diagram illustrating structures of compounds (3-54) to (3-65) as arylamine compounds represented by formula (3).
[Fig. 11] Fig. 11 is a diagram illustrating structures of compounds (3-66) to (3-74) as arylamine compounds represented by formula (3).
[Fig. 12] Fig. 12 is a diagram illustrating structures of compounds (A-1) to (A-15) as first host compounds represented by formula (Host-A).
[Fig. 13] Fig. 13 is a diagram illustrating structures of compounds (A-16) to (A-28) as first host compounds represented by formula (Host-A).
[Fig. 14] Fig. 14 is a diagram illustrating structures of compounds (A-29) to (A-43) as first host compounds represented by formula (Host-A).
[Fig. 15] Fig. 15 is a diagram illustrating structures of compounds (A-44) to (A-56) as first host compounds represented by formula (Host-A).
[Fig. 16] Fig. 16 is a diagram illustrating structures of compounds (A-57) to (A-69) as first host compounds represented by formula (Host-A).
[Fig. 17] Fig. 17 is a diagram illustrating structures of compounds (B-1) to (B-15) as second host compounds represented by formula (Host-B).
[Fig. 18] Fig. 18 is a diagram illustrating structures of compounds (B-16) to (B-27) as second host compounds represented by formula (Host-B).
[Fig. 19] Fig. 19 is a diagram illustrating structures of compounds (B-28) to (B-39) as second host compounds represented by formula (Host-B).
[Fig. 20] Fig. 20 is a diagram illustrating structures of compounds (B-40) to (B-52) as second host compounds represented by formula (Host-B).
[Fig. 21] Fig. 21 is a diagram illustrating structures of compounds (4-1) to (4-18) as metal complexes represented by formula (4).
[Fig. 22] Fig. 22 is a diagram illustrating structures of compounds (4-19) to (4-32) as metal complexes represented by formula (4).
[Fig. 23] Fig. 23 is a diagram illustrating structures of compounds (4-33) to (4-45) as metal complexes represented by formula (4).
[Fig. 24] Fig. 24 is a diagram illustrating structures of compounds (5-1) to (5-16) as arylamine compounds represented by formula (5).
[Fig. 25] Fig. 25 is a diagram illustrating structures of compounds (5-17) to (5-32) as arylamine compounds represented by formula (5).
[Fig. 26] Fig. 26 is a diagram illustrating structures of compounds (5-33) to (5-48) as arylamine compounds represented by formula (5).
[Fig. 27] Fig. 27 is a diagram illustrating structures of compounds (5-49) to (5-63) as arylamine compounds represented by formula (5).
[Fig. 28] Fig. 28 is a diagram illustrating a configuration of an organic EL device for Examples 13 to 22 and Comparative Examples 1 and 2.

### Description of Embodiments

Embodiments of the present invention will be described in detail below. First, various aspects of arylamine compounds and organic EL devices of the present invention will be described. Herein, the term "to" is a term indicating a range. For example, the description "5 to 10" means "from 5 to 10, inclusive," indicating a range including both the numerical values described before and after the term "to."

Concrete examples of the "aromatic hydrocarbon group", "aromatic heterocyclic group" or "fused polycyclic aromatic group" in the "substituted or unsubstituted aromatic hydrocarbon group", "substituted or unsubstituted aromatic heterocyclic group" or "substituted or unsubstituted fused polycyclic aromatic group" as represented by Ar₁ to Ar₈ in the aforementioned formulas (1), (2), and (3) may include a phenyl group, a biphenylyl group, a terphenylyl group, a naphthyl group, an anthracenyl group, a phenanthrenyl group, a fluorenyl group, an indenyl group, a pyrenyl group, a perylenyl group, a fluoranthenyl group, a triphenylenyl group, a pyridyl group, a pyrimidinyl group, a triazinyl group, a furyl group, a pyrrolyl group, a thienyl group, a selenonyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, a naphthyridinyl group, a phenanthrolinyl group, an acridinyl group, a carbolinyl group, etc.

Concrete examples of the "substituent" in the "substituted aromatic hydrocarbon group", "substituted aromatic heterocyclic group" or "substituted fused polycyclic aromatic group" as represented by Ar₁ to Ar₈ in the aforementioned formulas (1), (2), and (3) may include: a deuterium atom; a cyano group; a nitro group; halogen atoms, such as a fluorine atom, a chlorine atom, a bromine atom, or an iodine atom; linear or branched alkyl groups having 1 to 6 carbon atoms, such as a methyl group, an ethyl group, a n-propyl group, an isopropyl group, a n-butyl group, an isobutyl group, a tert-butyl group, a n-pentyl group, an isopentyl group, a neopentyl group, a n-hexyl group, etc.; linear or branched alkyloxy groups having 1 to 6 carbon atoms, such as a methyloxy group, an ethyloxy group, a propyloxy group, etc.; alkenyl groups, such as a vinyl group, an allyl group, etc.; aryloxy groups, such as a phenyloxy group, a tolyloxy group, etc.; arylalkyloxy groups, such as a benzyloxy group, a phenethyloxy group, etc.; aromatic hydrocarbon groups or fused polycyclic aromatic groups, such as a phenyl group, a biphenylyl group, a terphenylyl group, a naphthyl group, an anthracenyl group, a phenanthrenyl group, a fluorenyl group, an indenyl group, a pyrenyl group, a perylenyl group, a fluoranthenyl group, a triphenylenyl group, etc.; aromatic heterocyclic groups, such as a pyridyl group, a pyrimidinyl group, a triazinyl group, a thienyl group, a selenonyl group, a furyl group, a pyrrolyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, a carbolinyl group, etc.; arylvinyl groups, such as a styryl group, a naphthylvinyl group, etc.; and acyl groups, such as an acetyl group, a benzoyl group, etc. These substituents may further be substituted by any of the substituents given as examples above. Further, these substituents may be bonded to each other via a single bond, a substituted or unsubstituted methylene group, an oxygen atom, or a sulfur atom, to form a ring.

Concrete examples of the "linear or branched alkyl group having 1 to 6 carbon atoms", "cycloalkyl group having 5 to 10 carbon atoms" or "linear or branched alkenyl group having 2 to 6 carbon atoms" in the "linear or branched alkyl group having 1 to 6 carbon atoms and optionally having a substituent", "cycloalkyl group having 5 to 10 carbon atoms and optionally having a substituent" or "linear or branched alkenyl group having 2 to 6 carbon atoms and optionally having a substituent" as represented by R₁ to R₄ in the aforementioned formula (1) may include a methyl group, an ethyl group, a n-propyl group, an isopropyl group, a n-butyl group, an isobutyl group, a tert-butyl group, a n-pentyl group, an isopentyl group, a neopentyl group, a n-hexyl group, a cyclopentyl group, a cyclohexyl group, a 1-adamantyl group, a 2-adamantyl group, a vinyl group, an allyl group, an isopropenyl group, a 2-butenyl group, etc. These groups may be bonded to each other via a single bond, a substituted or unsubstituted methylene group, an oxygen atom, or a sulfur atom, to form a ring.

Concrete examples of the "substituent" in the "linear or branched alkyl group having 1 to 6 carbon atoms and having a substituent", "cycloalkyl group having 5 to 10 carbon atoms and having a substituent" or "linear or branched alkenyl group having 2 to 6 carbon atoms and having a substituent" as represented by R₁ to R₄ in the aforementioned formula (1) may include: a deuterium atom; a cyano group; a nitro group; halogen atoms, such as a fluorine atom, a chlorine atom, a bromine atom, or an iodine atom; linear or branched alkyloxy groups having 1 to 6 carbon atoms, such as a methyloxy group, an ethyloxy group, a propyloxy group, etc.; alkenyl groups, such as a vinyl group, an allyl group, etc.; aryloxy groups, such as a phenyloxy group, a tolyloxy group, etc.; arylalkyloxy groups, such as a benzyloxy group, a phenethyloxy group, etc.; aromatic hydrocarbon groups or fused polycyclic aromatic groups, such as a phenyl group, a biphenylyl group, a terphenylyl group, a naphthyl group, an anthracenyl group, a phenanthrenyl group, a fluorenyl group, an indenyl group, a pyrenyl group, a perylenyl group, a fluoranthenyl group, a triphenylenyl group, etc.; aromatic heterocyclic groups, such as a pyridyl group, a pyrimidinyl group, a triazinyl group, a thienyl group, a selenonyl group, a furyl group, a pyrrolyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, a carbolinyl group, etc. These substituents may further be substituted by any of the substituents given as examples above. Further, these substituents may be bonded to each other via a single bond, a substituted or unsubstituted methylene group, an oxygen atom, or a sulfur atom, to form a ring.

Concrete examples of the "linear or branched alkyloxy group having 1 to 6 carbon atoms" or "cycloalkyloxy group having 5 to 10 carbon atoms" in the "linear or branched alkyloxy group having 1 to 6 carbon atoms and optionally having a substituent" or "cycloalkyloxy group having 5 to 10 carbon atoms and optionally having a substituent" as represented by R₁ to R₄ in the aforementioned formula (1) may include a methyloxy group, an ethyloxy group, a n-propyloxy group, an isopropyloxy group, a n-butyloxy group, a tert-butyloxy group, a n-pentyloxy group, a n-hexyloxy group, a cyclopentyloxy group, a cyclohexyloxy group, a cycloheptyloxy group, a cyclooctyloxy group, a 1-adamantyloxy group, a 2-adamantyloxy group, etc. These groups may be bonded to each other via a single bond, a substituted or unsubstituted methylene group, an oxygen atom, or a sulfur atom, to form a ring.

Examples of the "substituent" in the "linear or branched alkyloxy group having 1 to 6 carbon atoms and having a substituent" or "cycloalkyloxy group having 5 to 10 carbon atoms and having a substituent" as represented by R₁ to R₄ in the aforementioned formula (1) may include the aforementioned examples given for the "substituent" in the "linear or branched alkyl group having 1 to 6 carbon atoms and having a substituent", "cycloalkyl group having 5 to 10 carbon atoms and having a substituent" or "linear or branched alkenyl group having 2 to 6 carbon atoms and having a substituent" as represented by R₁ to R₄ in the aforementioned general formula (1), and they may take similar forms/configurations as those described above.

Examples of the "aromatic hydrocarbon group", "aromatic heterocyclic group" or "fused polycyclic aromatic group" in the "substituted or unsubstituted aromatic hydrocarbon group", "substituted or unsubstituted aromatic heterocyclic group" or "substituted or unsubstituted fused polycyclic aromatic group" as represented by R₁ to R₄ in the aforementioned formula (1) may include the aforementioned examples given for the "aromatic hydrocarbon group", "aromatic heterocyclic group" or "fused polycyclic aromatic group" in the "substituted or unsubstituted aromatic hydrocarbon group", "substituted or unsubstituted aromatic heterocyclic group" or "substituted or unsubstituted fused polycyclic aromatic group" as represented by Ar₁ to Ar₈ in the aforementioned formulas (1), (2), and (3). These groups may be bonded to each other via a single bond, a substituted or unsubstituted methylene group, an oxygen atom, or a sulfur atom, to form a ring. Further, these groups may have a substituent, and examples of the substituent may include the aforementioned examples given for the "substituent" in the "substituted aromatic hydrocarbon group", "substituted aromatic heterocyclic group" or "substituted fused polycyclic aromatic group" as represented by Ar₁ to Ar₈ in the aforementioned formulas (1), (2), and (3), and they may take similar forms/configurations as those described above.

Concrete examples of the "aryloxy group" in the "substituted or unsubstituted aryloxy group" as represented by R₁ to R₄ in the aforementioned formula (1) may include a phenyloxy group, a biphenylyloxy group, a terphenylyloxy group, a naphthyloxy group, an anthracenyloxy group, a phenanthrenyloxy group, a fluorenyloxy group, an indenyloxy group, a pyrenyloxy group, a perylenyloxy group, etc. These groups may be bonded to each other via a single bond, a substituted or unsubstituted methylene group, an oxygen atom, or a sulfur atom, to form a ring. Further, these groups may have a substituent, and examples of the substituent may include the aforementioned examples given for the "substituent" in the "substituted aromatic hydrocarbon group", "substituted aromatic heterocyclic group" or "substituted fused polycyclic aromatic group" as represented by Ar₁ to Ar₈ in the aforementioned formulas (1), (2), and (3), and they may take similar forms/configurations as those described above.

One of R₁ to R₄ and Ar₁ in the aforementioned formula (1) is a linking group as a bonding site. Stated differently, one of R₁ to R₄ and Ar₁ in the aforementioned formula (1) is a linking group for bonding the group represented by the formula (1) and another structure in the arylamine compound having the group represented by the formula (1). In cases where one of R₁ to R₄ in the formula (1) is the linking group as the bonding site, R₁ to R₄ may be a single bond.

In the present invention, from the viewpoint of increasing the refractive index and the extinction coefficient, it is preferable that Ar₁ in the formula (1) is a "substituted or unsubstituted aromatic hydrocarbon group" or "substituted or unsubstituted fused polycyclic aromatic group", more preferably a phenyl group or a biphenyl group, and further preferably a phenyl group.

R₁ to R₄ in the formula (1) are preferably a hydrogen atom or a deuterium atom, and from the viewpoint of synthesis, are more preferably a hydrogen atom.

From the viewpoint of increasing the refractive index and the extinction coefficient, it is preferable that the arylamine compound having a group represented by the formula (1) is an arylamine compound represented by the aforementioned formula (2) or (3). It is also preferable that Ar₁ in the formula (1) is bonded to the nitrogen atom in the formula (2) or (3).

At least one of Ar₂ to Ar₄ in the formula (2) is a group represented by the formula (1), and it is preferable that two of Ar₂ to Ar₄ each independently represent a group represented by the formula (1). Further, at least one of Ar₅ to Ar₈ in the formula (3) is a group represented by the formula (1), and it is preferable that two of Ar₅ to Ar₈ each independently represent a group represented by the formula (1), and it is even preferable that Ar₅ and Ar₈ each independently represent a group represented by the formula (1).

Among Ar₂ to Ar₈ in the formulas (2) and (3), it is preferable that groups other than the group(s) represented by the formula (1) are preferably a "substituted or unsubstituted aromatic hydrocarbon group" or "substituted or unsubstituted fused polycyclic aromatic group", and more preferably a substituted or unsubstituted phenyl group or a substituted or unsubstituted biphenyl group. Examples of substituents of the phenyl group and biphenyl group may preferably include a phenyl group, a naphthyl group, and a phenanthrenyl group. Concrete examples of preferred groups may include groups represented by the following formulas.

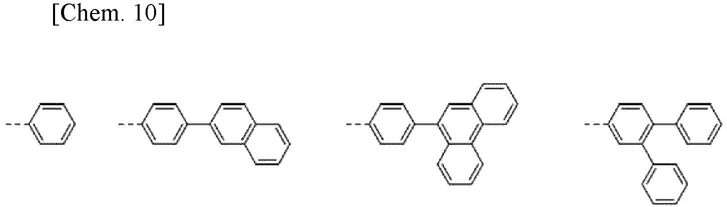

(In each formula, the broken line represents a bonding site.)

L₁ in the aforementioned formula (3) is preferably a substituted or unsubstituted biphenylene group, or a substituted or unsubstituted terphenylene group, and from the viewpoint of synthesis, is more preferably an unsubstituted biphenylene group or an unsubstituted terphenylene group.

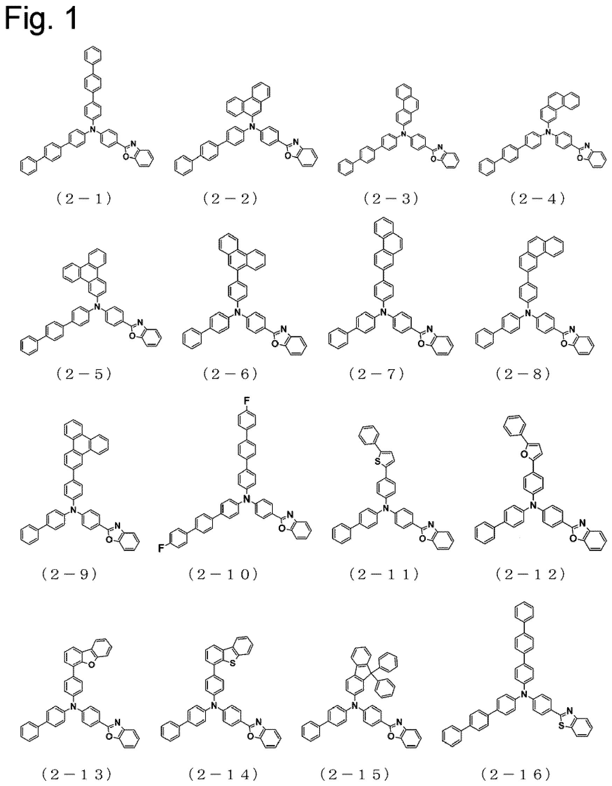

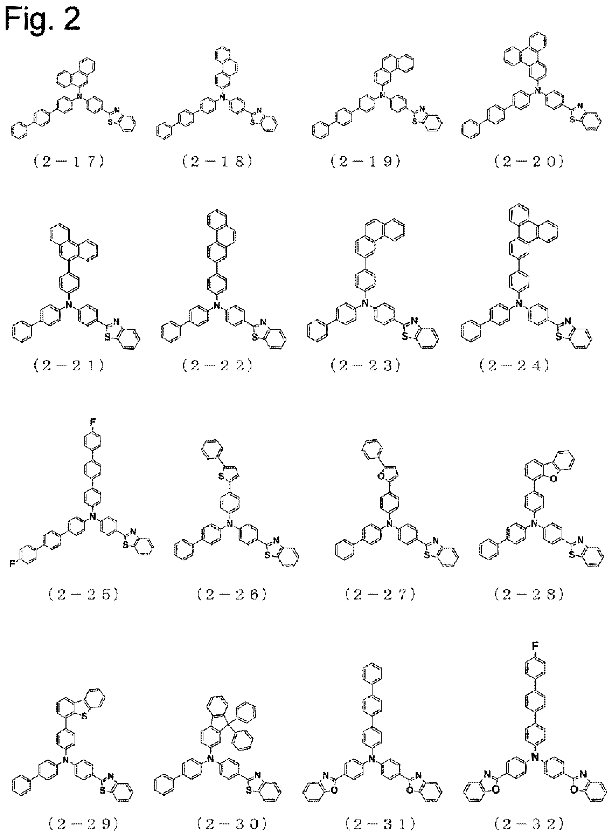

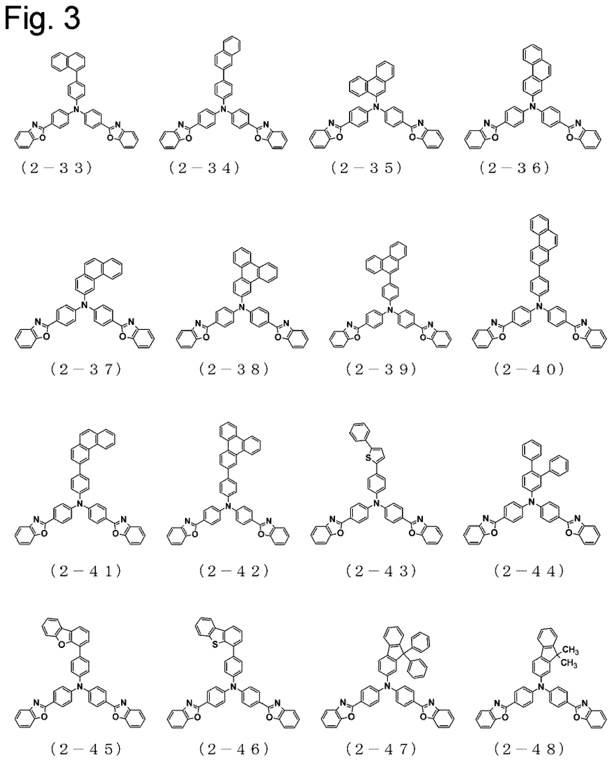

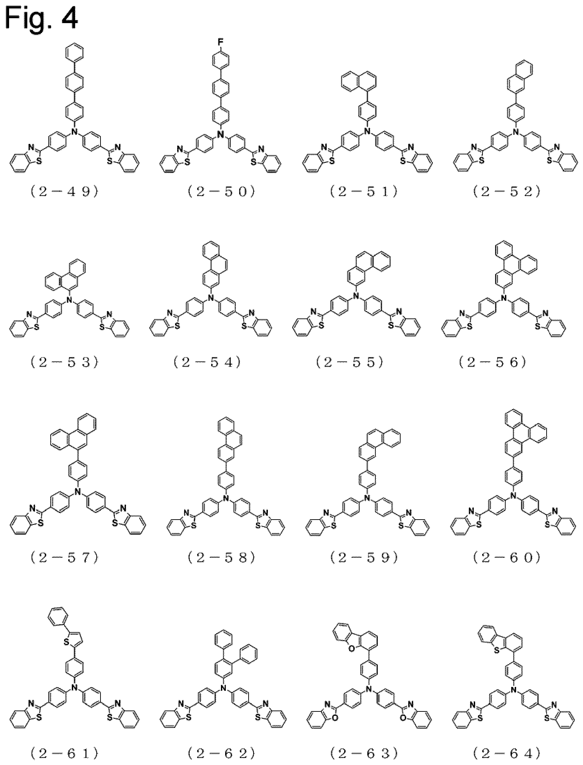

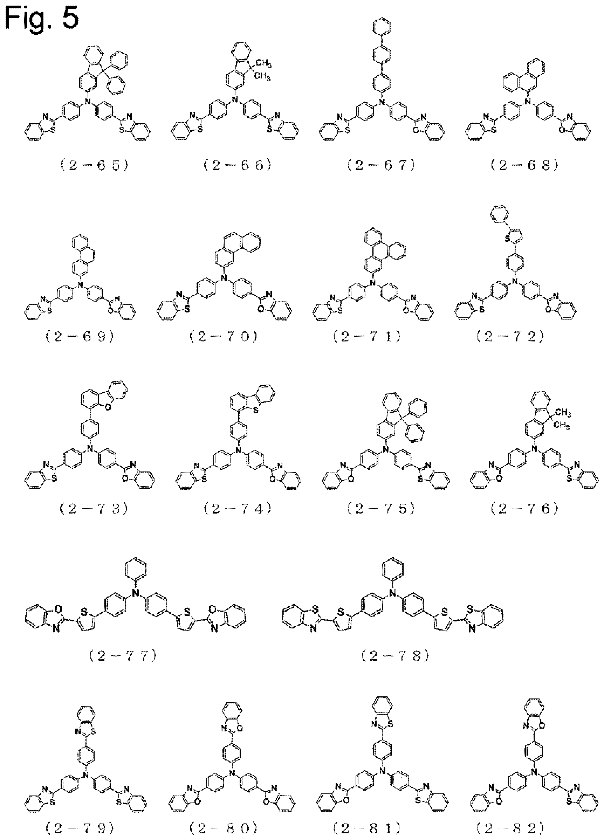

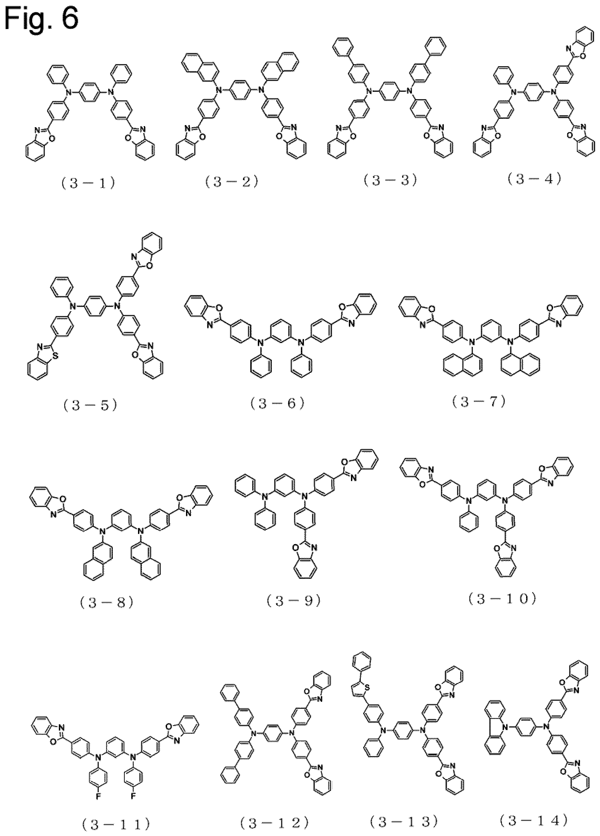

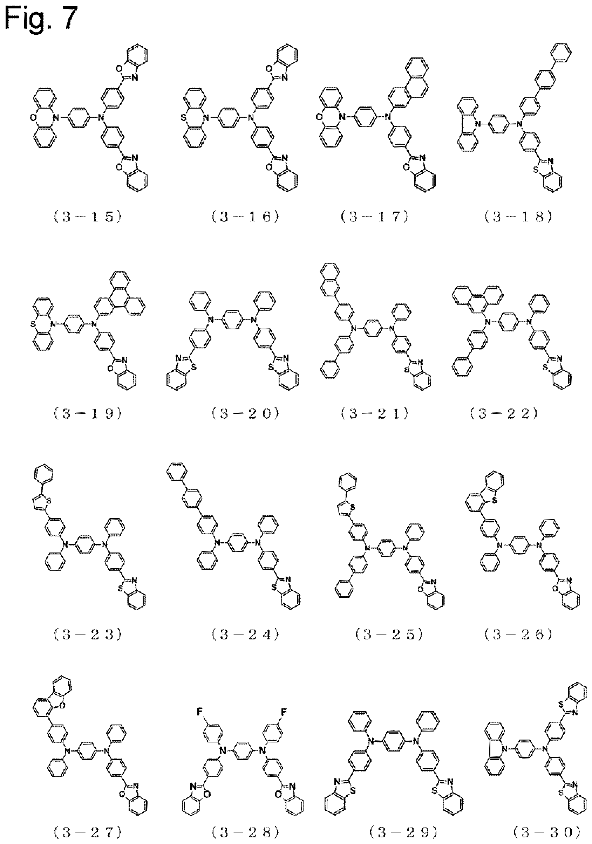

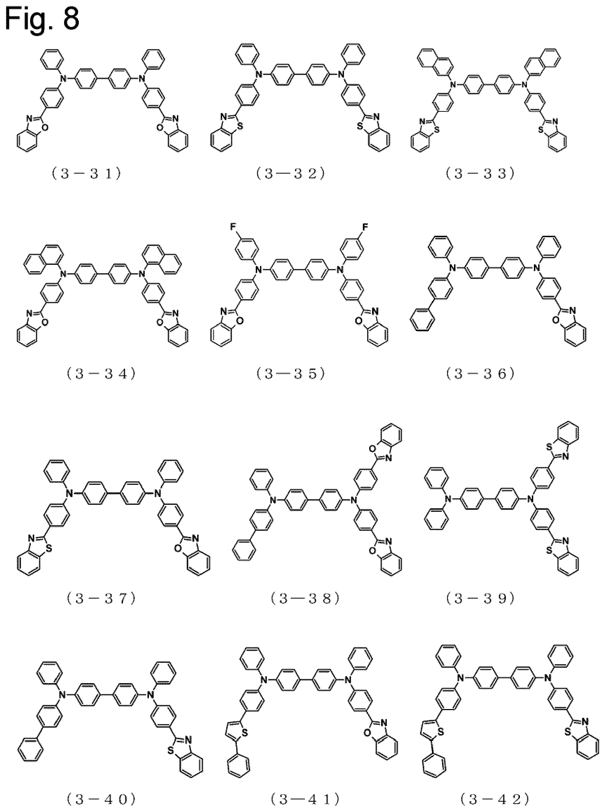

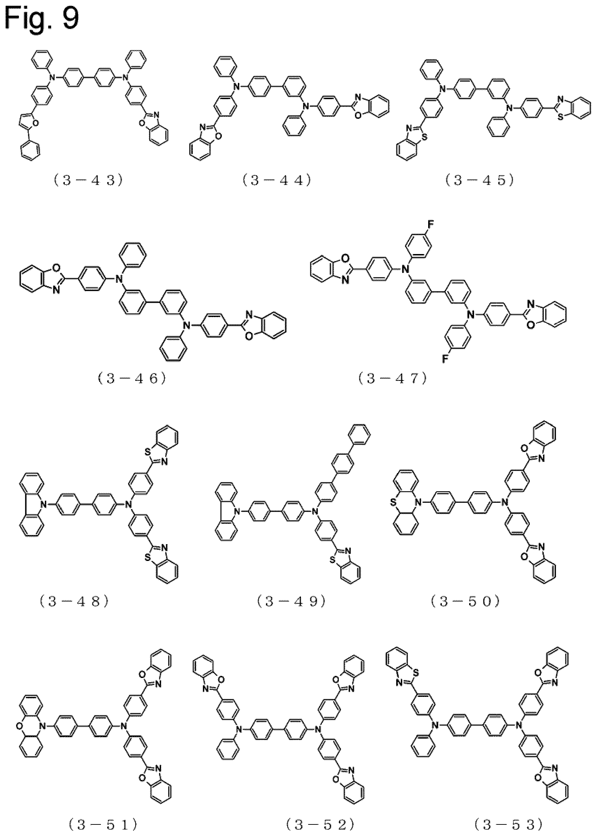

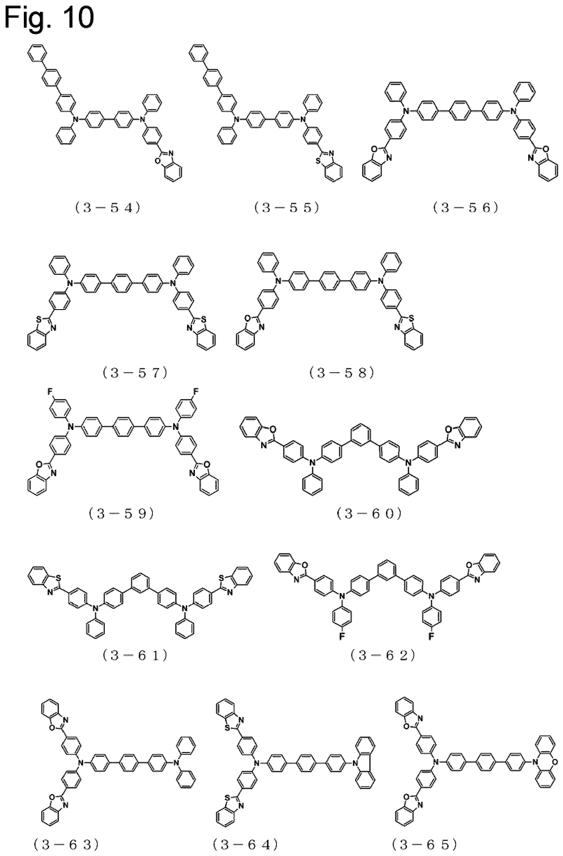

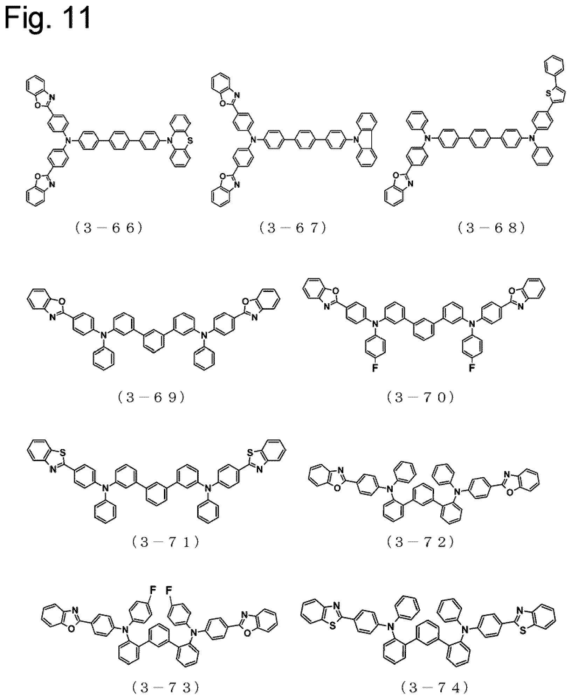

Figs. 1 to 11 illustrate concrete examples of preferred compounds among arylamine compounds represented by the formula (1) that may be suitably used for the organic EL device of the present invention. Note, however, that the compounds are not limited to the illustrated compounds.

Concrete examples of the "alkyl group having 1 to 15 carbon atoms" in the "substituted or unsubstituted alkyl group having 1 to 15 carbon atoms" as represented by R₅ to R₁₀ and Ra in the formula (Host-A) may include a methyl group, an ethyl group, a propyl group, an isopropyl group, a n-butyl group, a s-butyl group, an isobutyl group, a t-butyl group, a n-pentyl group, a n-hexyl group, a n-heptyl group, a n-octyl group, a hydroxymethyl group, a 1-hydroxyethyl group, a 2-hydroxyethyl group, a 2-hydroxyisobutyl group, a 1,2-dihydroxyethyl group, a 1,3-dihydroxyisopropyl group, a 2,3-dihydroxy-t-butyl group, a 1,2,3-trihydroxypropyl group, a chloromethyl group, a 1-chloroethyl group, a 2-chloroethyl group, a 2-chloroisobutyl group, a 1,2-dichloroethyl group, a 1,3-dichloroisopropyl group, a 2,3-dichloro-t-butyl group, a 1,2,3-trichloropropyl group, a bromomethyl group, a 1-bromoethyl group, a 2-bromoethyl group, a 2-bromoisobutyl group, a 1,2-dibromoethyl group, a 1,3-dibromoisopropyl group, a 2,3-dibromo-t-butyl group, a 1,2,3-tribromopropyl group, an iodomethyl group, a 1-iodoethyl group, a 2-iodoethyl group, a 2-iodoisobutyl group, a 1,2-diiodoethyl group, a 1,3-diiodoisopropyl group, a 2,3-diiodo-t-butyl group, a 1,2,3-triiodopropyl group, an aminomethyl group, a 1-aminoethyl group, a 2-aminoethyl group, a 2-aminoisobutyl group, a 1,2-diaminoethyl group, a 1,3-diaminoisopropyl group, a 2,3-diamino-t-butyl group, a 1,2,3-triaminopropyl group, a cyanomethyl group, a 1-cyanoethyl group, a 2-cyanoethyl group, a 2-cyanoisobutyl group, a 1,2-dicyanoethyl group, a 1,3-dicyanoisopropyl group, a 2,3-dicyano-t-butyl group, a 1,2,3-tricyanopropyl group, a nitromethyl group, a 1-nitroethyl group, a 2-nitroethyl group, a 2-nitroisobutyl group, a 1,2-dinitroethyl group, a 1,3-dinitroisopropyl group, a 2,3-dinitro-t-butyl group, a 1,2,3-trinitropropyl group, a cyclopropyl group, a cyclobutyl group, a cyclopentyl group, a cyclohexyl group, a 4-methylcyclohexyl group, a 1-adamantyl group, a 2-adamantyl group, a 1-norbornyl group, a 2-norbornyl group, etc.

Examples of the "substituent" in the "substituted or unsubstituted alkyl group having 1 to 15 carbon atoms" as represented by R₅ to R₁₀ and Ra in the formula (Host-A) may include the aforementioned examples given for the "substituent" in the "linear or branched alkyl group having 1 to 6 carbon atoms and having a substituent", "cycloalkyl group having 5 to 10 carbon atoms and having a substituent" or "linear or branched alkenyl group having 2 to 6 carbon atoms and having a substituent" as represented by R₁ to R₄ in the aforementioned formula (1), and they may take similar forms/configurations as those described above.

Concrete examples of the "substituted or unsubstituted aryl group having 6 to 15 carbon atoms" as represented by R₅ to R₁₀ and Ra in the formula (Host-A) may include a phenyl group, a biphenylyl group, a 9,9'-dimethylfluorenyl group, a 1-naphthyl group, a 2-naphthyl group, a fluorophenyl group, a difluorophenyl group, a trifluorophenyl group, a tetrafluorophenyl group, a pentafluorophenyl group, a toluyl group, a nitrophenyl group, a cyanophenyl group, a fluorobiphenylyl group, a nitrobiphenylyl group, a cyanobiphenyl group, a cyanonaphthyl group, a nitronaphthyl group, a fluoronaphthyl group, etc. These groups may be bonded to each other via a single bond, a substituted or unsubstituted methylene group, an oxygen atom, or a sulfur atom, to form a ring.

Examples of the "substituent" in the "substituted or unsubstituted aryl group having 6 to 12 ring-forming carbon atoms" as represented by R₅ to R₁₀ and Ra in the formula (Host-A) may include the aforementioned examples given for the "substituent" in the "substituted aromatic hydrocarbon group", "substituted aromatic heterocyclic group" or "substituted fused polycyclic aromatic group" as represented by Ar₁ to Ar₈ in the aforementioned formulas (1), (2), and (3), and they may take similar forms/configurations as those described above.

Concrete examples of the "arylene group having 6 to 30 ring-forming carbon atoms" or "heteroarylene group having 5 to 30 ring-forming carbon atoms" in the "substituted or unsubstituted arylene group having 6 to 30 ring-forming carbon atoms" or "substituted or unsubstituted heteroarylene group having 5 to 30 ring-forming carbon atoms" as represented by L₁ and L₂ in the formula (Host-A) may include a phenylene group, a biphenylene group, a terphenylene group, a quaterphenylene group, a quinquephenylene group, a naphthylene group, an anthrylene group, a phenanthrylene group, a fluorenylene group, an indenylene group, a pyrenylene group, an acetonaphtenylene group, a fluoranthenylene group, a triphenylenylene group, a pyridylene group, a pyranylene group, a quinolylene group, an isoquinolylene group, a benzofuranylene group, a benzothienylene group, an indolylene group, a carbazolylene group, a benzoxazolylene group, a benzothiazolylene group, a quinoxalylene group, a benzimidazolylene group, a pyrazolylene group, a dibenzofuranylene group, a dibenzothienylene group, etc.

Examples of the "substituent" in the "substituted or unsubstituted arylene group having 6 to 30 ring-forming carbon atoms" or "substituted or unsubstituted heteroarylene group having 5 to 30 ring-forming carbon atoms" as represented by L₁ and L₂ in the formula (Host-A) may include the aforementioned examples given for the "substituent" in the "substituted aromatic hydrocarbon group", "substituted aromatic heterocyclic group" or "substituted fused polycyclic aromatic group" as represented by Ar₁ to Ar₈ in the aforementioned formulas (1), (2), and (3), and they may take similar forms/configurations as those described above.

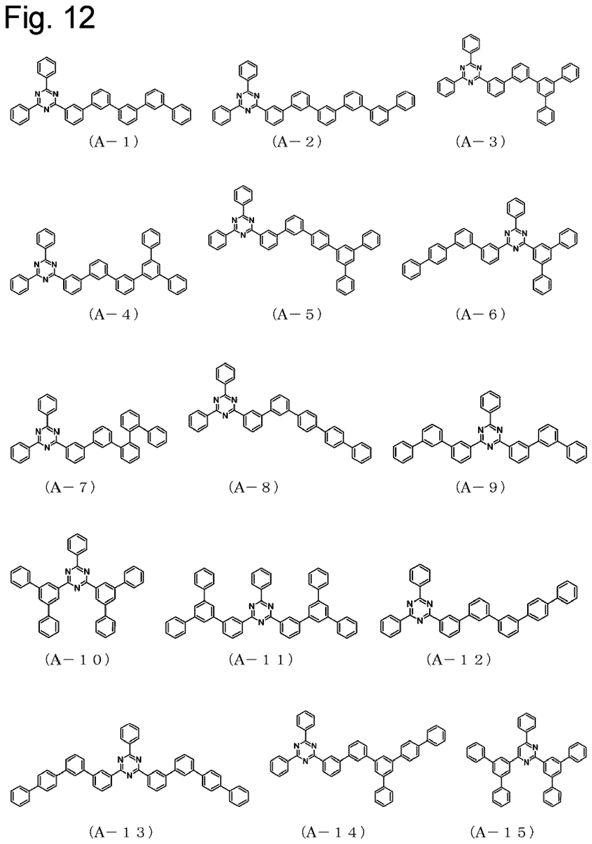

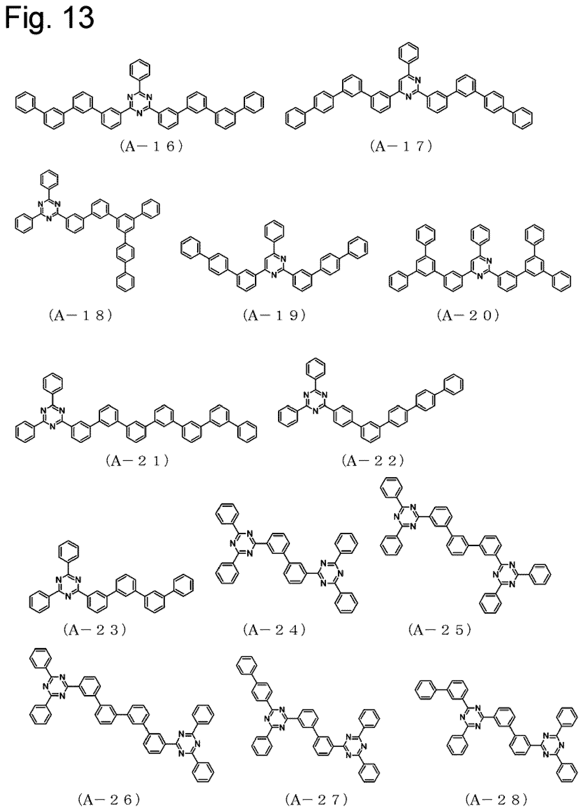

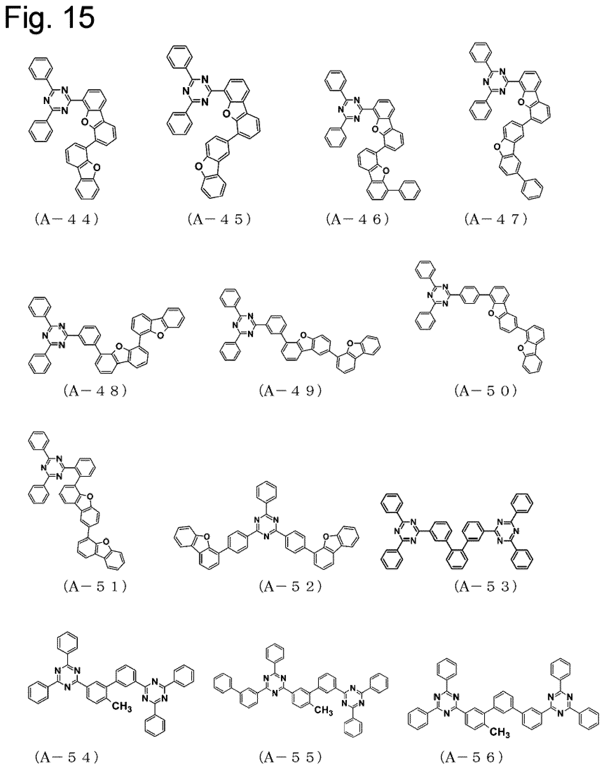

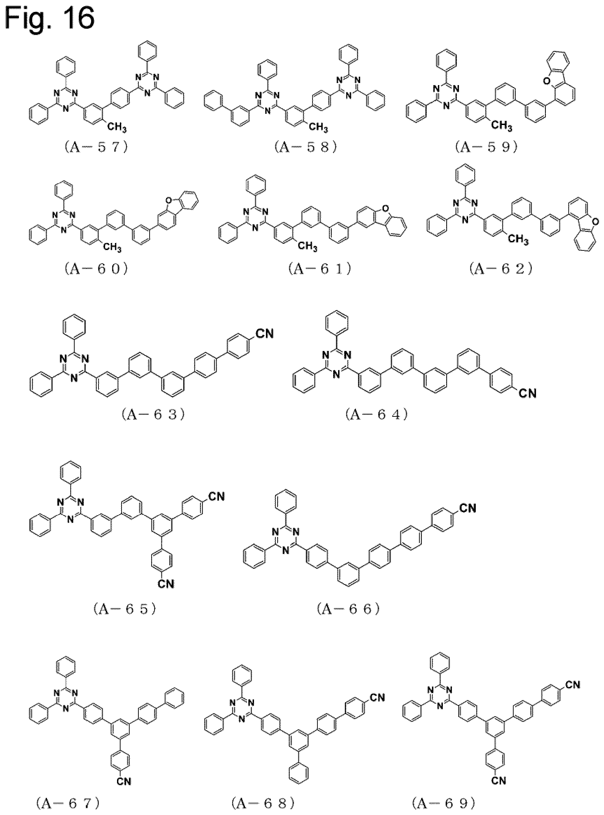

Figs. 12 to 16 illustrate concrete examples of compounds among first host compounds represented by the formula (Host-A) that may be suitably used for organic EL devices. Note, however, that the compounds are not limited to the illustrated compounds.

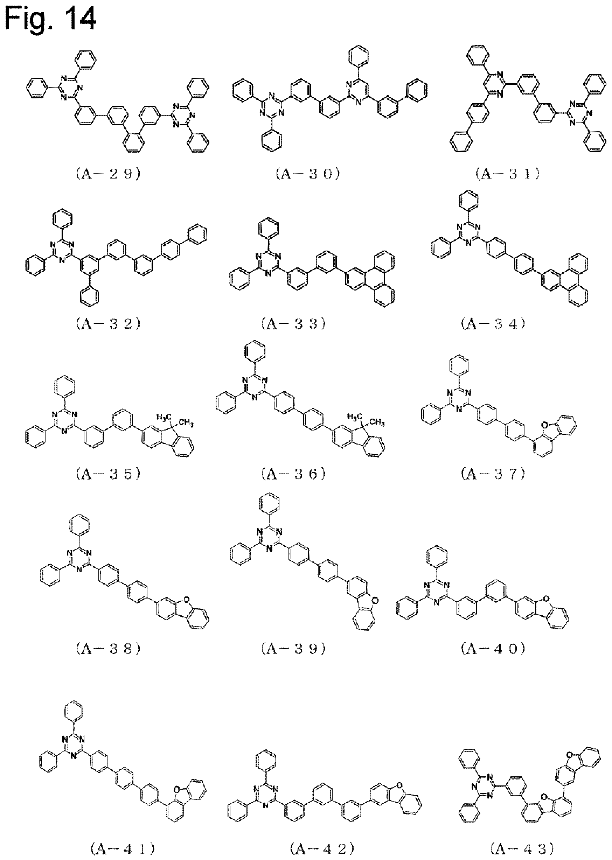

In the present invention, from the viewpoint of luminous efficiency and power efficiency, compound (A-43) illustrated in Fig. 14 is used as Host-A.

Concrete examples of the "aryl group having 6 to 30 carbon atoms" or "heteroaryl group having 5 to 30 carbon atoms" in the "substituted or unsubstituted aryl group having 6 to 30 carbon atoms" or "substituted or unsubstituted heteroaryl group having 5 to 30 carbon atoms" as represented by Ar₉ and Ar₁₀ in the formula (Host-B) may include a phenyl group, a biphenylyl group, a terphenylyl group, a naphthyl group, a triphenylene group, an anthracenyl group, a phenanthrenyl group, a fluorenyl group, an indenyl group, a pyrenyl group, a perylenyl group, a fluoranthenyl group, a triphenylenyl group, a pyridyl group, a pyrimidinyl group, a triazinyl group, a furyl group, a pyrrolyl group, a thienyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, a naphthyridinyl group, a phenanthrolinyl group, an acridinyl group, a carbolinyl group, etc. These groups may be bonded to each other via a single bond, a substituted or unsubstituted methylene group, an oxygen atom, or a sulfur atom, to form a ring.

Examples of the "substituent" in the "substituted or unsubstituted aryl group having 6 to 30 carbon atoms" or "substituted or unsubstituted heteroaryl group having 5 to 30 carbon atoms" as represented by Ar₉ and Ar₁₀ in the formula (Host-B) may include the aforementioned examples given for the "substituent" in the "substituted aromatic hydrocarbon group", "substituted aromatic heterocyclic group" or "substituted fused polycyclic aromatic group" as represented by Ar₁ to Ar₈ in the aforementioned formulas (1), (2), and (3), and they may take similar forms/configurations as those described above.

Concrete examples of the "alkyl group having 1 to 15 carbon atoms" as represented by R₁₁ to R₁₆ in the formula (Host-B) may include a methyl group, an ethyl group, a propyl group, an isopropyl group, a n-butyl group, a s-butyl group, an isobutyl group, a t-butyl group, a n-pentyl group, a n-hexyl group, a n-heptyl group, a n-octyl group, a hydroxymethyl group, a 1-hydroxyethyl group, a 2-hydroxyethyl group, a 2-hydroxyisobutyl group, a 1,2-dihydroxyethyl group, a 1,3-dihydroxyisopropyl group, a 2,3-dihydroxy-t-butyl group, a 1,2,3-trihydroxypropyl group, a chloromethyl group, a 1-chloroethyl group, a 2-chloroethyl group, a 2-chloroisobutyl group, a 1,2-dichloroethyl group, a 1,3-dichloroisopropyl group, a 2,3-dichloro-t-butyl group, a 1,2,3-trichloropropyl group, a bromomethyl group, a 1-bromoethyl group, a 2-bromoethyl group, a 2-bromoisobutyl group, a 1,2-dibromoethyl group, a 1,3-dibromoisopropyl group, a 2,3-dibromo-t-butyl group, a 1,2,3-tribromopropyl group, an iodomethyl group, a 1-iodoethyl group, a 2-iodoethyl group, a 2-iodoisobutyl group, a 1,2-diiodoethyl group, a 1,3-diiodoisopropyl group, a 2,3-diiodo-t-butyl group, a 1,2,3-triiodopropyl group, an aminomethyl group, a 1-aminoethyl group, a 2-aminoethyl group, a 2-aminoisobutyl group, a 1,2-diaminoethyl group, a 1,3-diaminoisopropyl group, a 2,3-diamino-t-butyl group, a 1,2,3-triaminopropyl group, a cyanomethyl group, a 1-cyanoethyl group, a 2-cyanoethyl group, a 2-cyanoisobutyl group, a 1,2-dicyanoethyl group, a 1,3-dicyanoisopropyl group, a 2,3-dicyano-t-butyl group, a 1,2,3-tricyanopropyl group, a nitromethyl group, a 1-nitroethyl group, a 2-nitroethyl group, a 2-nitroisobutyl group, a 1,2-dinitroethyl group, a 1,3-dinitroisopropyl group, a 2,3-dinitro-t-butyl group, a 1,2,3-trinitropropyl group, a cyclopropyl group, a cyclobutyl group, a cyclopentyl group, a cyclohexyl group, a 4-methylcyclohexyl group, a 1-adamantyl group, a 2-adamantyl group, a 1-norbornyl group, a 2-norbornyl group, etc.

Examples of the "substituent" in the "alkyl group having 1 to 15 carbon atoms" as represented by R₁₁ to R₁₆ in the formula (Host-B) may include the aforementioned examples given for the "substituent" in the "linear or branched alkyl group having 1 to 6 carbon atoms and having a substituent", "cycloalkyl group having 5 to 10 carbon atoms and having a substituent" or "linear or branched alkenyl group having 2 to 6 carbon atoms and having a substituent" as represented by R₁ to R₄ in the aforementioned formula (1), and they may take similar forms/configurations as those described above.

Examples of the "substituted or unsubstituted aryl group having 6 to 30 ring-forming carbon atoms" or "substituted or unsubstituted heteroaryl group having 5 to 30 ring-forming carbon atoms" as represented by R₁₁ to R₁₆ in the formula (Host-B) may include a phenyl group, a biphenylyl group, a terphenylyl group, a naphthyl group, an anthracenyl group, a phenanthrenyl group, a fluorenyl group, an indenyl group, a pyrenyl group, a perylenyl group, a fluoranthenyl group, a triphenylenyl group, a pyridyl group, a pyrimidinyl group, a triazinyl group, a furyl group, a pyrrolyl group, a thienyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, a naphthyridinyl group, a phenanthrolinyl group, an acridinyl group, a carbolinyl group, etc. These groups may be bonded to each other via a single bond, a substituted or unsubstituted methylene group, an oxygen atom, or a sulfur atom, to form a ring.

Examples of the "substituent" in the "substituted or unsubstituted aryl group having 6 to 30 ring-forming carbon atoms" or "substituted or unsubstituted heteroaryl group having 5 to 30 ring-forming carbon atoms" as represented by R₁₁ to R₁₆ in the formula (Host-B) may include the aforementioned examples given for the "substituent" in the "substituted aromatic hydrocarbon group", "substituted aromatic heterocyclic group" or "substituted fused polycyclic aromatic group" as represented by Ar₁ to Ar₈ in the aforementioned formulas (1), (2), and (3), and they may take similar forms/configurations as those described above.

Concrete examples of the "arylene group having 6 to 30 carbon atoms" or "heteroarylene group having 5 to 30 carbon atoms" in the "substituted or unsubstituted arylene group having 6 to 30 carbon atoms" or "substituted or unsubstituted heteroarylene group having 5 to 30 carbon atoms" as represented by Y₁ and Y₂ in the formula (Host-B) may include a phenylene group, a biphenylene group, a terphenylene group, a naphthylene group, an anthrylene group, a phenanthrylene group, a fluorenylene group, an indenylene group, a pyrenylene group, an acetonaphtenylene group, a fluoranthenylene group, a triphenylenylene group, a pyridylene group, a pyranylene group, a quinolylene group, an isoquinolylene group, a benzofuranylene group, a benzothienylene group, an indolylene group, a carbazolylene group, a benzoxazolylene group, a benzothiazolylene group, a quinoxalylene group, a benzimidazolylene group, a pyrazolylene group, a dibenzofuranylene group, a dibenzothienylene group, etc. Particularly preferred among the above are a phenylene group, a biphenylene group, or a pyrenylene group.

Examples of the "substituent" in the "substituted or unsubstituted aryl group having 6 to 30 carbon atoms" or "substituted or unsubstituted heteroaryl group having 5 to 30 carbon atoms" as represented by Y₁ and Y₂ in the formula (Host-B) may include the aforementioned examples given for the "substituent" in the "substituted aromatic hydrocarbon group", "substituted aromatic heterocyclic group" or "substituted fused polycyclic aromatic group" as represented by Ar₁ to Ar₈ in the aforementioned formulas (1), (2), and (3), and they may take similar forms/configurations as those described above.

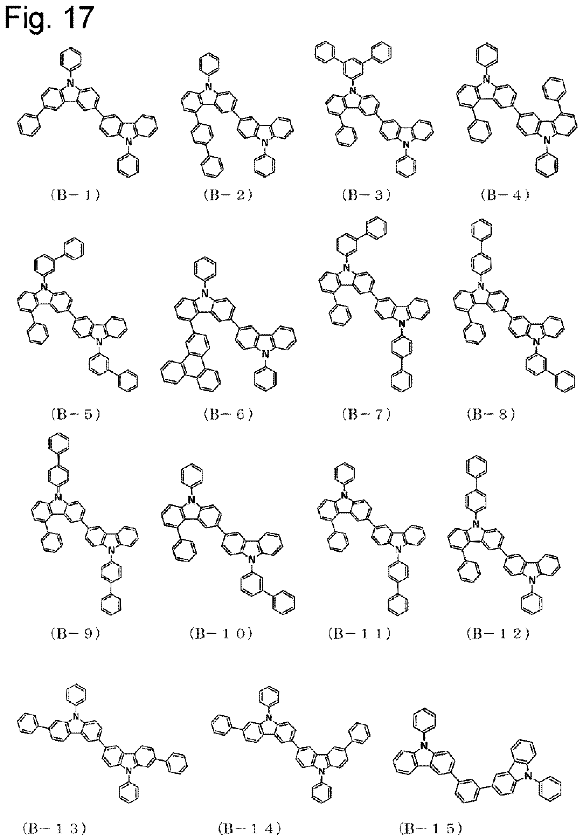

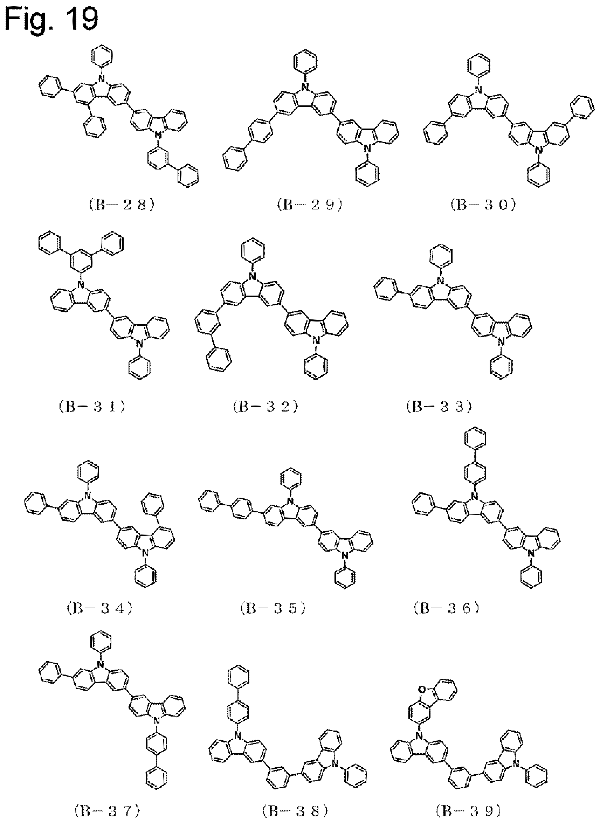

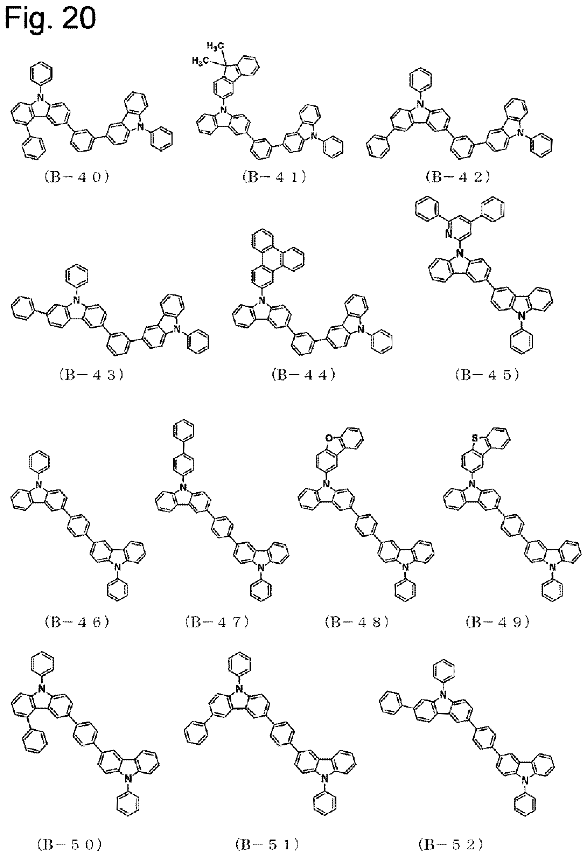

Figs. 17 to 20 illustrate concrete examples of compounds among second host compounds represented by the formula (Host-B) that may be suitably used for organic EL devices. Note, however, that the compounds are not limited to the illustrated compounds.

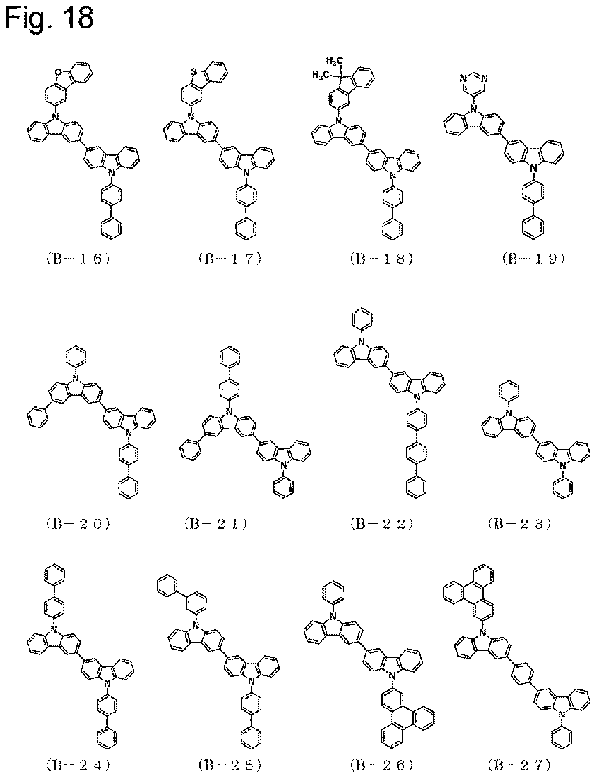

In the present invention, from the viewpoint of luminous efficiency and power efficiency, compound (B-25) illustrated in Fig. 18 is used as Host-B.

Concrete examples of the "linear or branched alkyl group having 1 to 6 carbon atoms", "cycloalkyl group having 5 to 10 carbon atoms" or "linear or branched alkenyl group having 2 to 6 carbon atoms" in the "linear or branched alkyl group having 1 to 6 carbon atoms and optionally having a substituent", "cycloalkyl group having 5 to 10 carbon atoms and optionally having a substituent" or "linear or branched alkenyl group having 2 to 6 carbon atoms and optionally having a substituent" as represented by R₁₇ to R₃₂ in the formula (4) may include a methyl group, an ethyl group, a n-propyl group, an isopropyl group, a n-butyl group, an isobutyl group, a tert-butyl group, a n-pentyl group, an isopentyl group, a neopentyl group, a n-hexyl group, a cyclopentyl group, a cyclohexyl group, a 1-adamantyl group, a 2-adamantyl group, a vinyl group, an allyl group, an isopropenyl group, a 2-butenyl group, etc. These groups may be bonded to each other via a single bond, a substituted or unsubstituted methylene group, an oxygen atom, or a sulfur atom, to form a ring.

Examples of the "substituent" in the "linear or branched alkyl group having 1 to 6 carbon atoms and optionally having a substituent", "cycloalkyl group having 5 to 10 carbon atoms and optionally having a substituent" or "linear or branched alkenyl group having 2 to 6 carbon atoms and optionally having a substituent" as represented by R₁₇ to R₃₂ in the formula (4) may include the aforementioned examples given for the "substituent" in the "linear or branched alkyl group having 1 to 6 carbon atoms and having a substituent", "cycloalkyl group having 5 to 10 carbon atoms and having a substituent" or "linear or branched alkenyl group having 2 to 6 carbon atoms and having a substituent" as represented by R₁ to R₄ in the aforementioned formula (1), and they may take similar forms/configurations as those described above.

Concrete examples of the "linear or branched alkyloxy group having 1 to 6 carbon atoms" or "cycloalkyloxy group having 5 to 10 carbon atoms" in the "linear or branched alkyloxy group having 1 to 6 carbon atoms and optionally having a substituent" or "cycloalkyloxy group having 5 to 10 carbon atoms and optionally having a substituent" as represented by R₁₇ to R₃₂ in the formula (4) may include a methyloxy group, an ethyloxy group, a n-propyloxy group, an isopropyloxy group, a n-butyloxy group, a tert-butyloxy group, a n-pentyloxy group, a n-hexyloxy group, a cyclopentyloxy group, a cyclohexyloxy group, a cycloheptyloxy group, a cyclooctyloxy group, a 1-adamantyloxy group, a 2-adamantyloxy group, etc.

Examples of the "substituent" in the "linear or branched alkyloxy group having 1 to 6 carbon atoms and having a substituent" or "cycloalkyloxy group having 5 to 10 carbon atoms and having a substituent" as represented by R₁₇ to R₃₂ in the formula (4) may include the aforementioned examples given for the "substituent" in the "linear or branched alkyl group having 1 to 6 carbon atoms and having a substituent", "cycloalkyl group having 5 to 10 carbon atoms and having a substituent" or "linear or branched alkenyl group having 2 to 6 carbon atoms and having a substituent" as represented by R₁ to R₄ in the aforementioned formula (1), and they may take similar forms/configurations as those described above.

Concrete examples of the "aryloxy group" in the "substituted or unsubstituted aryloxy group" as represented by R₁₇ to R₃₂ in the formula (4) may include a phenyloxy group, a biphenylyloxy group, a terphenylyloxy group, a naphthyloxy group, an anthracenyloxy group, a phenanthrenyloxy group, a fluorenyloxy group, an indenyloxy group, a pyrenyloxy group, a perylenyloxy group, etc. These groups may be bonded to each other via a single bond, a substituted or unsubstituted methylene group, an oxygen atom, or a sulfur atom, to form a ring.

Examples of the "substituent" in the "substituted or unsubstituted aryloxy group" as represented by R₁₇ to R₃₂ in the formula (4) may include the aforementioned examples given for the "substituent" in the "substituted or unsubstituted aryloxy group" as represented by R₁ to R₄ in the aforementioned formula (1), and they may take similar forms/configurations as those described above.

Concrete examples of the "aromatic hydrocarbon group", "aromatic heterocyclic group" or "fused polycyclic aromatic group" in the "substituted or unsubstituted aromatic hydrocarbon group", "substituted or unsubstituted aromatic heterocyclic group" or "substituted or unsubstituted fused polycyclic aromatic group" as represented by R₁₇ to R₃₂ in the formula (4) may include a phenyl group, a naphthyl group, an anthracenyl group, a phenanthryl group, a naphthacenyl group, a pyrenyl group, a biphenylyl group, a p-terphenyl group, a m-terphenyl group, a chrysenyl group, a triphenylenyl group, a perylenyl group, an indenyl group, a furanyl group, a thiophenyl group, a pyrrolyl group, a pyrazolyl group, an imidazolyl group, a triazolyl group, an oxazolyl group, a thiazolyl group, an oxadiazolyl group, a thiadiazolyl group, a pyridyl group, a pyrimidinyl group, a pyrazinyl group, a triazinyl group, a benzofuranyl group, a benzothiophenyl group, a benzimidazolyl group, an indolyl group, a quinolinyl group, an isoquinolinyl group, a quinazolinyl group, a quinoxalinyl group, a naphthyridinyl group, a benzoxazinyl group, a benzothiazinyl group, an acridinyl group, a phenazinyl group, a phenothiazinyl group, a phenoxazinyl group, etc. These groups may be bonded to each other via a single bond, a substituted or unsubstituted methylene group, an oxygen atom, or a sulfur atom, to form a ring.

Examples of the "substituent" in the "substituted or unsubstituted aromatic hydrocarbon group", "substituted or unsubstituted aromatic heterocyclic group", or "substituted or unsubstituted fused polycyclic aromatic group" as represented by R₁₇ to R₃₂ in the formula (4) may include the aforementioned examples given for the "substituent" in the "substituted aromatic hydrocarbon group", "substituted aromatic heterocyclic group" or "substituted fused polycyclic aromatic group" as represented by Ar₁ to Ar₈ in the aforementioned formulas (1), (2), and (3), and they may take similar forms/configurations as those described above.

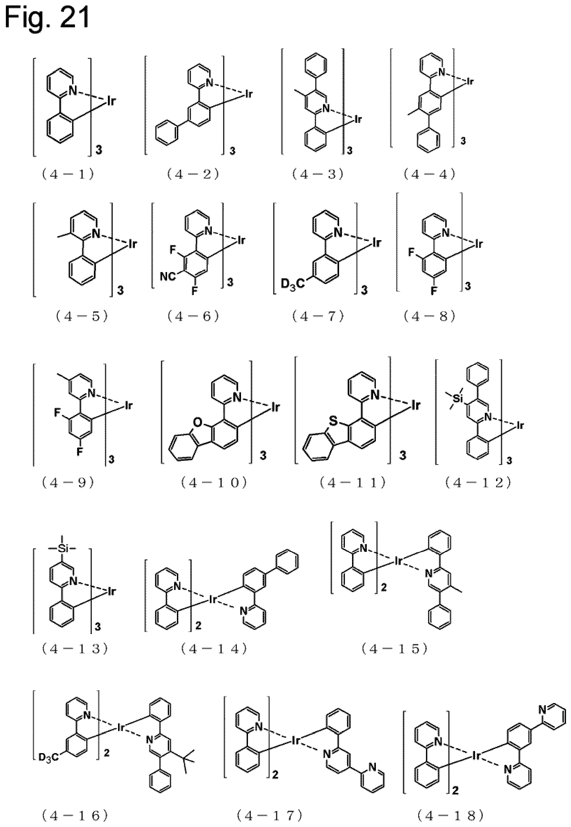

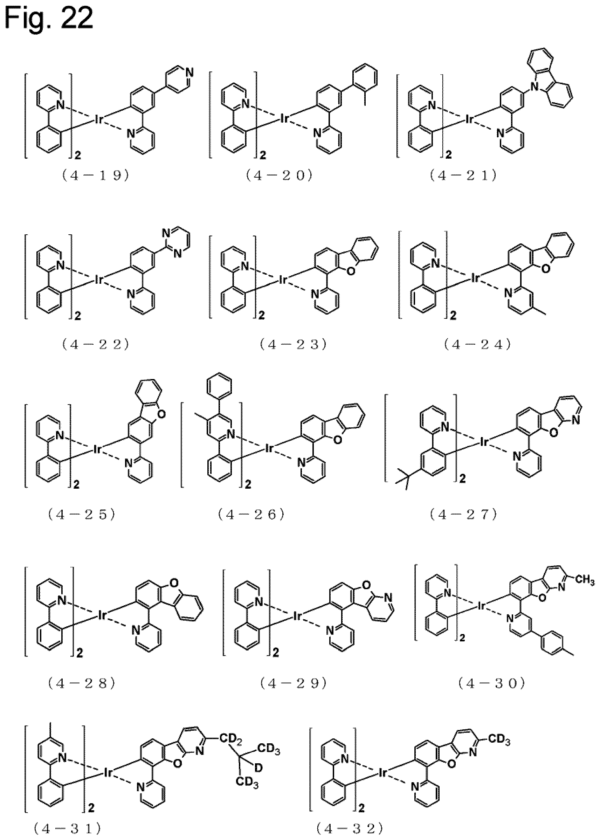

Figs. 21 to 23 illustrate concrete examples of compounds among metal complexes represented by the formula (4) that may be suitably used for the organic EL devices. Note, however, that the compounds are not limited to the illustrated compounds.

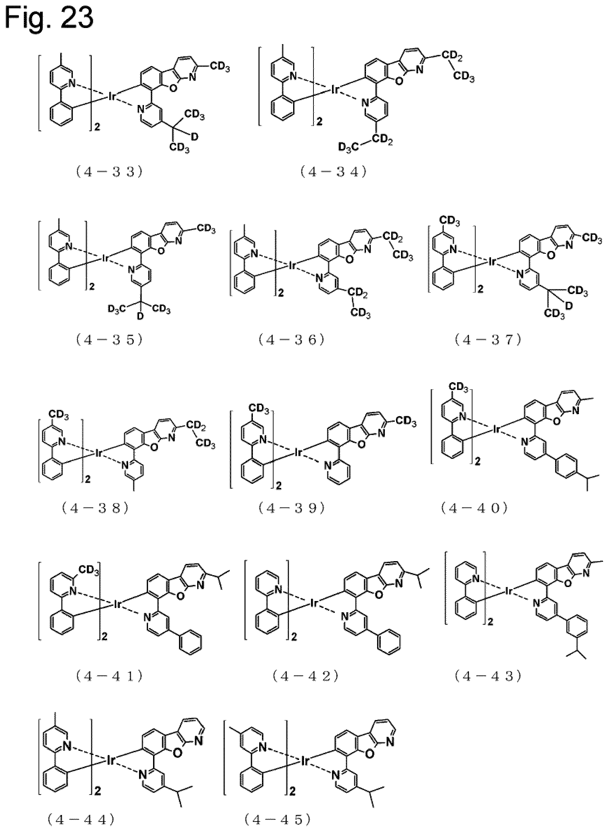

In the present invention, from the viewpoint of luminous efficiency and power efficiency, compound (4-35) illustrated in Fig. 23 is as a phosphorescent dopant.

Concrete examples of the "aryl group having 6 to 30 carbon atoms" or "heteroaryl group having 5 to 30 carbon atoms" in the "substituted or unsubstituted aryl group having 6 to 30 carbon atoms" or "substituted or unsubstituted heteroaryl group having 5 to 30 carbon atoms" as represented by Ar₁₁ and Ar₁₂ in the formula (5) may include a phenyl group, a biphenylyl group, a terphenylyl group, a naphthyl group, an anthracenyl group, a phenanthrenyl group, a fluorenyl group, an indenyl group, a pyrenyl group, a perylenyl group, a fluoranthenyl group, a triphenylenyl group, a pyridyl group, a pyrimidinyl group, a triazinyl group, a furyl group, a pyrrolyl group, a thienyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, a naphthyridinyl group, a phenanthrolinyl group, an acridinyl group, a carbolinyl group, etc. These groups may be bonded to each other via a single bond, a substituted or unsubstituted methylene group, an oxygen atom, or a sulfur atom, to form a ring.

Examples of the "substituent" in the "substituted or unsubstituted aryl group having 6 to 30 carbon atoms" or "substituted or unsubstituted heteroaryl group having 5 to 30 carbon atoms" as represented by Ar₁₁ and Ar₁₂ in the formula (5) may include the aforementioned examples given for the "substituent" in the "substituted aromatic hydrocarbon group", "substituted aromatic heterocyclic group" or "substituted fused polycyclic aromatic group" as represented by Ar₁ to Ar₈ in the aforementioned formulas (1), (2), and (3), and they may take similar forms/configurations as those described above.

Concrete examples of the "alkyl group having 1 to 15 carbon atoms" in the "substituted or unsubstituted alkyl group having 1 to 15 carbon atoms" as represented by R₃₃ to R₃₉ in the formulas (5) and (6) may include a methyl group, an ethyl group, a propyl group, an isopropyl group, a n-butyl group, a s-butyl group, an isobutyl group, a t-butyl group, a n-pentyl group, a n-hexyl group, a n-heptyl group, a n-octyl group, a hydroxymethyl group, a 1-hydroxyethyl group, a 2-hydroxyethyl group, a 2-hydroxyisobutyl group, a 1,2-dihydroxyethyl group, a 1,3-dihydroxyisopropyl group, a 2,3-dihydroxy-t-butyl group, a 1,2,3-trihydroxypropyl group, a chloromethyl group, a 1-chloroethyl group, a 2-chloroethyl group, a 2-chloroisobutyl group, a 1,2-dichloroethyl group, a 1,3-dichloroisopropyl group, a 2,3-dichloro-t-butyl group, a 1,2,3-trichloropropyl group, a bromomethyl group, a 1-bromoethyl group, a 2-bromoethyl group, a 2-bromoisobutyl group, a 1,2-dibromoethyl group, a 1,3-dibromoisopropyl group, a 2,3-dibromo-t-butyl group, a 1,2,3-tribromopropyl group, an iodomethyl group, a 1-iodoethyl group, a 2-iodoethyl group, a 2-iodoisobutyl group, a 1,2-diiodoethyl group, a 1,3-diiodoisopropyl group, a 2,3-diiodo-t-butyl group, a 1,2,3-triiodopropyl group, an aminomethyl group, a 1-aminoethyl group, a 2-aminoethyl group, a 2-aminoisobutyl group, a 1,2-diaminoethyl group, a 1,3-diaminoisopropyl group, a 2,3-diamino-t-butyl group, a 1,2,3-triaminopropyl group, a cyanomethyl group, a 1-cyanoethyl group, a 2-cyanoethyl group, a 2-cyanoisobutyl group, a 1,2-dicyanoethyl group, a 1,3-dicyanoisopropyl group, a 2,3-dicyano-t-butyl group, a 1,2,3-tricyanopropyl group, a nitromethyl group, a 1-nitroethyl group, a 2-nitroethyl group, a 2-nitroisobutyl group, a 1,2-dinitroethyl group, a 1,3-dinitroisopropyl group, a 2,3-dinitro-t-butyl group, a 1,2,3-trinitropropyl group, a cyclopropyl group, a cyclobutyl group, a cyclopentyl group, a cyclohexyl group, a 4-methylcyclohexyl group, a 1-adamantyl group, a 2-adamantyl group, a 1-norbornyl group, a 2-norbornyl group, etc.

Examples of the "substituent" in the "substituted or unsubstituted alkyl group having 1 to 15 carbon atoms" as represented by R₃₃ to R₃₉ in the formulas (5) and (6) may include the aforementioned examples given for the "substituent" in the "linear or branched alkyl group having 1 to 6 carbon atoms and having a substituent", "cycloalkyl group having 5 to 10 carbon atoms and having a substituent" or "linear or branched alkenyl group having 2 to 6 carbon atoms and having a substituent" as represented by R₁ to R₄ in the aforementioned formula (1), and they may take similar forms/configurations as those described above.

Concrete examples of the "aryl group having 6 to 12 carbon atoms" in the "substituted or unsubstituted aryl group having 6 to 12 carbon atoms" as represented by R₃₃ to R₃₉ in the formulas (5) and (6) may include a phenyl group, a biphenylyl group, a 1-naphthyl group, a 2-naphthyl group, a fluorophenyl group, a difluorophenyl group, a trifluorophenyl group, a tetrafluorophenyl group, a pentafluorophenyl group, a toluyl group, a nitrophenyl group, a cyanophenyl group, a fluorobiphenylyl group, a nitrobiphenylyl group, a cyanobiphenyl group, a cyanonaphthyl group, a nitronaphthyl group, a fluoronaphthyl group, etc. These groups may be bonded to each other via a single bond, a substituted or unsubstituted methylene group, an oxygen atom, or a sulfur atom, to form a ring.

Examples of the "substituent" in the "substituted or unsubstituted aryl group having 6 to 12 carbon atoms" as represented by R₃₃ to R₃₉ in the formulas (5) and (6) may include the aforementioned examples given for the "substituent" in the "substituted aromatic hydrocarbon group", "substituted aromatic heterocyclic group" or "substituted fused polycyclic aromatic group" as represented by Ar₁ to Ar₈ in the aforementioned formulas (1), (2), and (3), and they may take similar forms/configurations as those described above.

In the present invention, from the viewpoint of hole transportability, it is preferable that Ar₁₁ and Ar₁₂ in the formula (5) are an unsubstituted biphenyl group, an unsubstituted terphenyl group, an unsubstituted triphenylenyl group, an unsubstituted phenanthrenyl group, a fluorenyl group having a substituent, or a thienyl group having a substituent, and more preferably an unsubstituted biphenyl group or a fluorenyl group having a substituent. It is preferable that the substituent of the fluorenyl group and thienyl group is a methyl group or a phenyl group, and it is even preferable that the substituent of the fluorenyl group is a methyl group.

It is preferable that R₃₃ to R₃₇ in the formulas (5) and (6) are a hydrogen atom or a deuterium atom, and from the viewpoint of synthesis, is more preferably a hydrogen atom. It is preferable that R₃₈ and R₃₉ in the formula (6) is a methyl group.

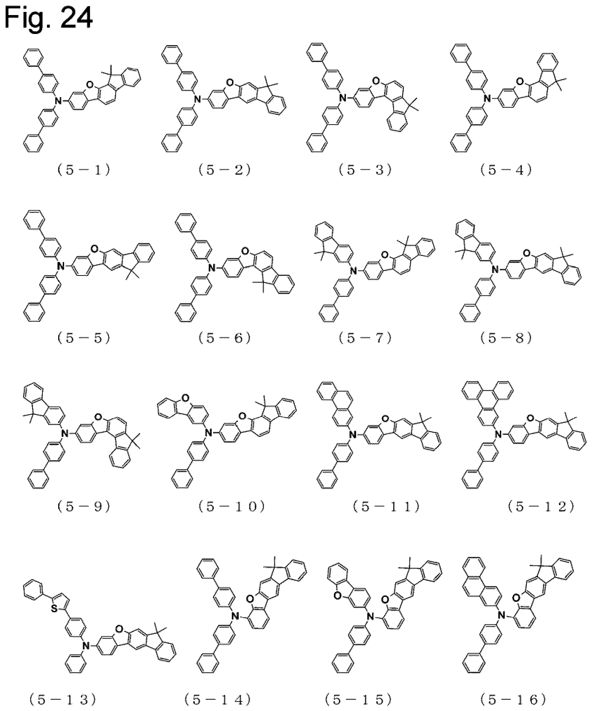

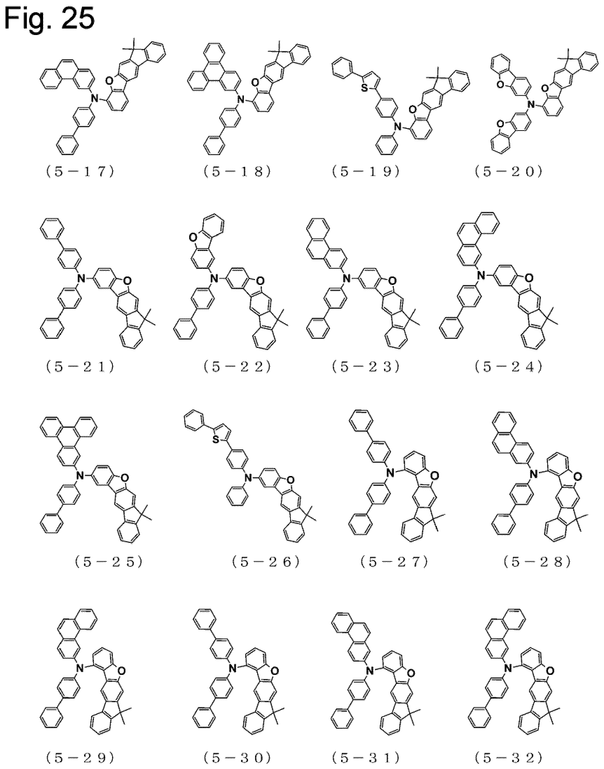

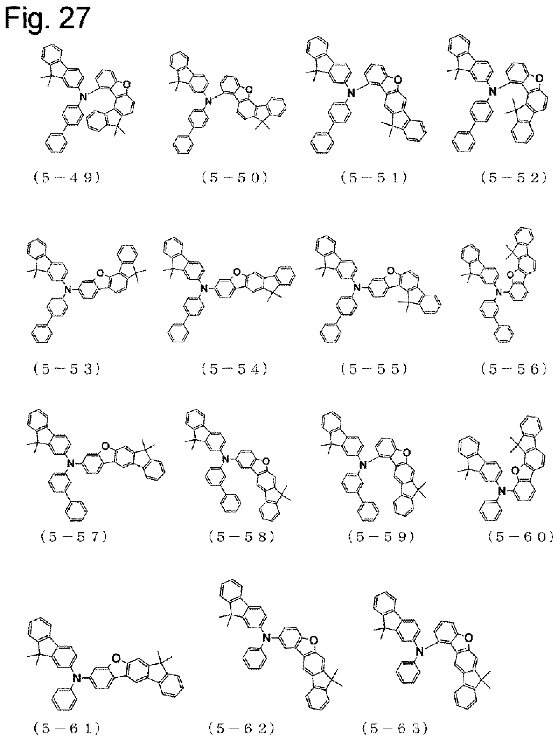

Figs. 24 to 27 illustrate concrete examples of preferred compounds among arylamine compounds represented by the formula (5) that may be suitably used for the organic EL device of the present invention. Note, however, that the compounds are not limited to the illustrated compounds.

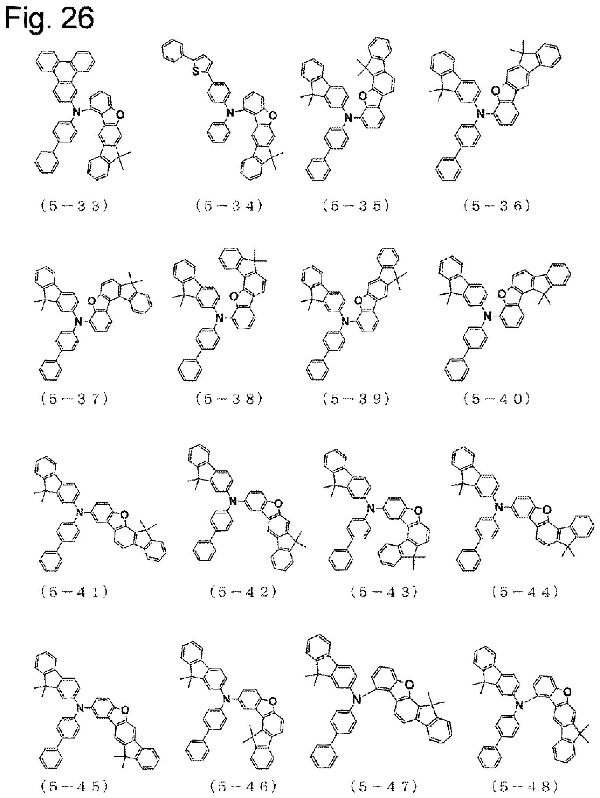

In the present invention, from the viewpoint of hole transportability and thin film stability, compound (5-48) illustrated in Fig. 26 is preferable, although the compound does not constitute a limitation.

The arylamine compound having a group represented by the formula (1) according to the present invention is a novel compound, having a higher refractive index within an optical wavelength range of 450 nm to 650 nm than conventional capping materials, and having an action of more suitably improving the light extraction efficiency of organic EL devices particularly in cases where green and/or and red phosphorescent light-emitting materials are included.

Further, the arylamine compound having a group represented by the formula (1) according to the present invention has a higher extinction coefficient within an optical wavelength range of 400 nm to 410 nm than conventional capping materials, and thus has an action of preventing damage to devices due to sunlight and can therefore achieve high durability.

The organic EL device according to the present invention employs an arylamine compound having a group represented by the formula (1), i.e., having a benzazole ring structure, that has a higher refractive index than conventional capping materials, has excellent light absorption coefficient, and excellent amorphous properties, and can thus achieve high efficiency and high durability.

The benzazole derivative in formula (1) which is the main skeleton of the compound can be synthesized according to known methods (see, for example, Non-Patent Literature 4). The synthesized halogenated benzazole derivative and an arylamine can be subjected to a copper-catalyzed or palladium-catalyzed coupling reaction, to synthesize an amine compound having a group represented by the formula (1) according to the present invention. Alternatively, the halogenated benzazole derivative can be made into a boronic acid derivative or a boronate ester derivative, and then subjected to a coupling reaction with a halogenated arylamine, to synthesize an arylamine compound having a group represented by the formula (1) according to the present invention (see, for example, Non-Patent Literatures 4 and 5).

The first host compound represented by the aforementioned formula (Host-A) can be synthesized according to known methods (see, for example, Patent Literatures 3, 4, and 8).

The second host compound represented by the aforementioned formula (Host-B) can be synthesized according to known methods (see, for example, Patent Literatures 3 and 4).

The metal complex represented by the aforementioned formula (4) can be synthesized according to known methods (see, for example, Patent Literatures 9 and 10).

The arylamine compound represented by the aforementioned formula (5) can be synthesized according to known methods (see, for example, Patent Literature 11).

Purification of the amine compound having a group represented by the formula (1), the first host compound represented by the formula (Host-A), the second host compound represented by the formula (Host-B), the metal complex represented by the formula (4), and the arylamine compound represented by the formula (5) can be achieved by such techniques as column chromatography purification, adsorption purification with silica gel, activated carbon, activated clay, etc., recrystallization or crystallization using a solvent, sublimation purification, or the like. Compound identification can be achieved by NMR analysis. Physical properties to be measured preferably include such values as the melting point, glass transition point (Tg), work function, or the like. The melting point serves as an index of vapor deposition characteristics. The glass transition point (Tg) serves as an index of stability in a thin-film state. The work function serves as an index of hole transportability and hole blockability.

As for compounds to be used in the organic EL device of the present invention, it is preferable to use compounds first purified by, for example, column chromatography purification, adsorption purification with silica gel, activated carbon, activated clay, etc., recrystallization or crystallization using a solvent, or the like, and then lastly purified by sublimation purification.

The melting point and glass transition point (Tg) can be measured with a high-sensitivity differential scanning calorimeter (DSC3100SA from Bruker AXS) using a powder.

For the amine compound having a group represented by the formula (1), it is preferable to measure its refractive index and extinction coefficient. The refractive index and extinction coefficient can be measured with a spectrometer (F10-RT-UV from Filmetrics) by preparing an 80-nm thin film on a silicon substrate.

For example, a structure of the organic EL device of the present invention may sequentially include, on a glass substrate, an anode, a hole injection layer, a first hole transport layer, a second hole transport layer, a light-emitting layer, an electron transport layer, a cathode, and a capping layer, in cases where the device is a top-emission-structure light-emitting device. In other examples: a hole injection layer may be provided between the anode and the hole transport layer; an electron blocking layer may be provided between the hole transport layer and the light-emitting layer; a hole blocking layer may be provided between the light-emitting layer and the electron transport layer; or an electron injection layer may be provided between the electron transport layer and the cathode. In such multilayer structures, a single organic layer may have functions of several layers; for example, a single organic layer may have: functions of the hole injection layer and the hole transport layer; functions of the hole transport layer and the electron blocking layer; functions of the hole blocking layer and the electron transport layer; or functions of the electron transport layer and the electron injection layer. Further, it is possible to stack two or more organic layers having the same function; for example, the structure may include: two hole transport layers; two light-emitting layers; two electron transport layers; or two capping layers.

The total thickness of the various layers of the organic EL device is preferably around 100 nm to 700 nm, more preferably around 150 nm to 300 nm. The thickness of the capping layer may be, for example, preferably 30 nm to 120 nm, more preferably 40 nm to 80 nm. In this range, excellent light extraction efficiency can be obtained. Note that the thickness of the capping layer can be varied as appropriate depending on, for example, the type of light-emitting material to be used in the light-emitting device, the thickness of the organic EL device excluding the capping layer, or the like.

For the material for the hole injection layer in the organic EL device of the present invention, it is possible to use: an arylamine compound including, in its molecule, three or more triphenylamine structures which are linked by a single bond or a divalent group containing no hetero atom, such as a starburst triphenylamine derivative or one of various triphenylamine tetramers; a porphyrin compound typified by copper phthalocyanine; an acceptor heterocyclic compound such as hexacyanoazatriphenylene; or a coating-type polymer material.

For the material for the first hole transport layer in the organic EL device of the present invention, it is preferable to use a benzidine derivative, such as N,N'-diphenyl-N,N'-di(m-tolyl)benzidine (abbreviated hereinbelow as "TPD"), N,N'-diphenyl-N,N'-di(α-naphthyl)benzidine (abbreviated hereinbelow as "NPD"), N,N,N',N'-tetrabiphenylylbenzidine, etc., or 1,1-bis[4-(di-4-tolylamino)phenyl]cyclohexane (abbreviated hereinbelow as "TAPC"), and particularly, an arylamine compound including, in its molecule, two triphenylamine structures which are linked by a single bond or a divalent group containing no hetero atom, such as N, N, N', N'-tetrabiphenylylbenzidine. It is also preferable to use an arylamine compound including, in its molecule, three or more triphenylamine structures which are linked by a single bond or a divalent group containing no hetero atom, such as one of various triphenylamine trimers and tetramers.

Further, for the material for the hole injection-transport layer, it is possible to use a coating-type polymer material, such as poly(3,4-ethylenedioxythiophene) (abbreviated hereinbelow as "PEDOT")/poly(styrene sulfonate) (abbreviated hereinbelow as "PSS").

For the material for the second hole transport layer in the organic EL device of the present invention, it is preferable to use an arylamine compound represented by the formula (5), but it is also possible to use a compound having an electron blocking action, such as: a carbazole derivative, such as 4,4',4"-tri(N-carbazolyl)triphenylamine (abbreviated hereinbelow as "TCTA"), 9,9-bis[4-(carbazol-9-yl)phenyl]fluorene, 1,3-bis(carbazol-9-yl)benzene (abbreviated hereinbelow as "mCP"), 2,2-bis(4-carbazol-9-ylphenyl)adamantane (abbreviated hereinbelow as "Ad-Cz"), etc.; or a compound containing a triarylamine structure and a triphenylsilyl group typified by 9-[4-(carbazol-9-yl)phenyl]-9-[4-(triphenylsilyl)phenyl]-9H-fluorene, etc.

For the material for the hole injection layer and the hole transport layer, it is possible to use: a material commonly used for such layers and p-doped with trisbromophenylamine hexachloroantimonate or a radialene derivative (see, for example, later-described compound (Acceptor-1) and Patent Literature 12); or a polymer compound having, as a partial structure thereof, a benzidine derivative structure such as TPD.

For the host in the light-emitting layer of the organic EL devices, it is possible to use a hole-transporting host material and an electron-transporting host material. For the hole-transporting host material, it is possible to use the second host compound represented by the aforementioned formula (Host-B), and/or a carbazole derivative such as 4,4'-di(N-carbazolyl)biphenyl (CBP), TCTA, mCP, etc. For the electron-transporting host material, it is possible to use the first host compound represented by the aforementioned formula (Host-A), and/or p-bis(triphenylsilyl)benzene (UGH2), 2,2',2"-(1,3,5-phenylene)-tris(1-phenyl-1H-benzimidazole) (TPBi), etc.

Generally, it is preferable to use at least two types of compounds including a first host compound having electron transportability and a second host compound having hole transportability. One type, or two or more types, of the second host compound(s) may be used. The weight ratio between the first host compound to the second host compound may be, for example, 1:10 to 10:1.

For the first host compound in the light-emitting layer of the organic EL devices, it is preferable to use a compound having a nitrogen-containing heteroaromatic ring structure represented by the aforementioned formula (Host-A). For the second host compound, it is preferable to use a compound having a carbazole ring structure represented by the aforementioned formula (Host-B).

Other than the aforementioned first host compound and second host compound, it is possible to further include one or more types of host compounds.

The phosphorescent dopant in the light-emitting layer of the organic EL device of the present invention is a metal complex represented by the formula (4-35). In organic EL devices not forming part of the invention, it is also possible to use an organometallic compound containing Pt, Os, Ti, Zr, Hf, Eu, Tb, Tm, Fe, Co, Ni, Ru, Rh, Pd, or a combination thereof. The dopant may be a red, green or blue dopant, enabling production of a high-performance organic EL device.

To avoid concentration quenching, doping of the host material(s) with a phosphorescent dopant is preferably performed by co-vapor deposition within a range of 1 to 30 wt.% with respect to the entire light-emitting layer.

For the material for the hole blocking layer in the organic EL device of the present invention, it is possible to use a compound having a hole blocking action, with examples including phenanthroline derivatives such as bathocuproine (abbreviated hereinbelow as "BCP"), metal complexes of a quinolinol derivative such as bis(2-methyl-8-quinolinolato))-4-phenylphenolato aluminum (III) (abbreviated hereinbelow as "BAlq"), various rare-earth complexes, triazole derivatives, triazine derivatives, pyrimidine derivatives, oxadiazole derivatives, benzazole derivatives, etc. These materials may also serve as materials for the electron transport layer.

For the material for the electron transport layer in the organic EL device of the present invention, it is possible to use a metal complex of a quinolinol derivative such as Alq₃, BAlq, etc., one of various metal complexes, a triazole derivative, a triazine derivative, a pyrimidine derivative, an oxadiazole derivative, a pyridine derivative, a benzimidazole derivative, a benzazole derivative, a thiadiazole derivative, an anthracene derivative, a carbodiimide derivative, a quinoxaline derivative, a pyridoindole derivative, a phenanthroline derivative, a silole derivative, etc.

For the material for the electron injection layer in the organic EL device of the present invention, it is possible to use an alkali metal salt such as lithium fluoride, cesium fluoride, etc., an alkaline-earth metal salt such as magnesium fluoride, etc., a metal complex of a quinolinol derivative such as quinolinol lithium, etc., a metal oxide such as aluminum oxide, etc. The electron injection layer may, however, be omitted by suitable selection of the electron transport layer and the cathode.

For the material for the electron injection layer and the electron transport layer, it is possible to use an organic compound commonly used for such layers and n-doped with a metal such as cesium, lithium fluoride, ytterbium, etc.

For the cathode in the organic EL device of the present invention, an electrode material having a low work function, such as aluminum, ytterbium, etc., or an alloy having an even lower work function, such as magnesium silver alloy, magnesium indium alloy, aluminum magnesium alloy, etc., may be used as the electrode material.

The capping layer in the organic EL device of the present invention contains an arylamine compound having a group represented by the formula (1).

It is preferable that the material constituting the capping layer has a greater refractive index than the adjacent electrode. The capping layer can improve the light extraction efficiency in the organic EL device, but this effect is more significant when the reflectance at the interface between the capping layer and a material in contact therewith is greater, because a greater optical coherence effect can be obtained. Thus, it is preferable that the refractive index of the material constituting the capping layer is greater than the refractive index of the adjacent electrode, and, although it is sufficient if the refractive index is 1.70 or greater within a wavelength range of 450 nm to 650 nm, it is more preferable that the refractive index is 1.80 or greater, and particularly preferably 1.85 or greater.

The aforementioned materials used for the various layers constituting the organic EL device of the present invention can form thin films by known methods, such as vapor deposition, spin coating, ink-jetting, etc.

These materials may each be formed into a film singly, or a plurality of types of materials may be mixed and formed into a film, and each may be used as a single layer. It is possible to form a laminate structure constituted by layers each formed singly by the respective materials, or a laminate structure constituted by layers formed by mixing the materials, or a laminate structure constituted by layers each formed singly by the respective materials and layers formed by mixing a plurality of types of materials.

The above described a top-emission-structure organic EL device, but the present invention is not limited thereto, and is similarly applicable to bottom-emission-structure organic EL devices, as well as dual-emission organic EL devices that emit light from both the top and bottom sides. In these cases, it is necessary that the electrode(s) located in the direction in which light exits from the light-emitting device toward outside be transparent or semi-transparent.

Embodiments of the present invention will be described in further detail below according to working examples. Note, however, that the present invention is not limited to the following examples so long as they do not go beyond the gist of the invention.

### Examples

### Example 1:

### Synthesis of bis-{4-(benzoxazol-2-yl)phenyl}-{4-(naphthalen-2-yl)phenyl}amine (2-34):

A reaction vessel was charged with 7.5 g of 4-(naphthalen-2-yl)phenyl-amine, 20.6 g of 2-(4-bromophenyl)benzoxazole, 9.9 g of sodium t-butoxide, and 150 ml of toluene, and was aerated with nitrogen gas for 30 minutes under ultrasonic irradiation. Then, 0.9 g of tris(dibenzylideneacetone)dipalladium(0) and 0.4 ml of a 50% (w/v) toluene solution of tri-(t-butyl)phosphine were added, and the mixture was stirred for 3 hours under reflux with heating. The mixture was left to cool to 80°C; then, silica gel was added thereto and the mixture was filtrated. The filtrate was concentrated, to obtain a crude product. The crude product was recrystallized with toluene, to obtain 6.3 g of a yellow powder of bis-{4-(benzoxazol-2-yl)phenyl}-{4-(naphthalen-2-yl)phenyl}amine (2-34) (yield: 30%).

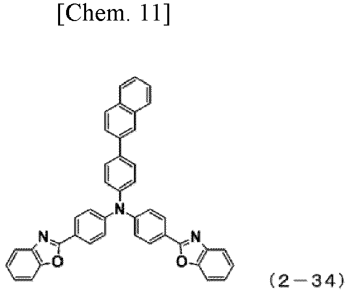

The structure of the obtained yellow powder was identified by NMR.

The following 27 hydrogen signals were detected with ¹H-NMR (CDCl₃).

δ (ppm) = 8.19-8.24 (4H), 8.09 (1H), 7.87-7.97 (3H), 7.73-7.83 (5H), 7.51-7.62 (4H), 7.32-7.42 (10H).

### Example 2:

### Synthesis of bis- { 4-(benzoxazol-2-yl)phenyl}-{4-(phenanthren-9-yl)phenyl } amine (2-39):

A reaction vessel was charged with 8.0 g of 4-(phenanthren-9-yl)phenyl-amine, 17.9 g of 2-(4-bromophenyl)benzoxazole, 8.6 g of sodium t-butoxide, and 160 ml of toluene, and was aerated with nitrogen gas for 30 minutes under ultrasonic irradiation. Then, 0.8 g of tris(dibenzylideneacetone)dipalladium(0) and 0.4 ml of a 50% (w/v) toluene solution of tri-(t-butyl)phosphine were added, and the mixture was stirred for 3 hours under reflux with heating. The mixture was left to cool to 80°C; then, silica gel was added thereto and the mixture was filtrated. The filtrate was concentrated, to obtain a crude product. The crude product was recrystallized with toluene, to obtain 15.0 g of a yellow powder of bis-{4-(benzoxazol-2-yl)phenyl}-{4-(phenanthren-9-yl)phenyl}amine (2-39) (yield: 77.0%).

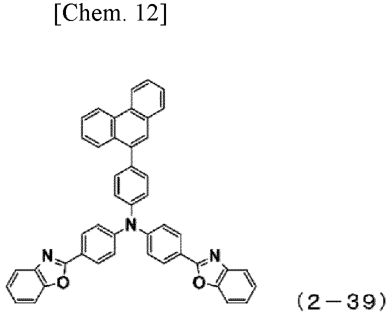

The structure of the obtained yellow powder was identified by NMR.

The following 29 hydrogen signals were detected with ¹H-NMR (CDCl₃).

δ (ppm) = 8.73-8.84 (2H), 8.24-8.27 (4H), 8.07-8.11 (1H), 7.92-7.97 (1H), 7.57-7.84 (11H), 7.35-7.43 (10H).

### Example 3:

### Synthesis of bis-{4-(benzoxazol-2-yl)phenyl}-([1,1',2',1"]terphenyl-4'-yl)-amine(2-44):

A reaction vessel was charged with 5.6 g of ([1,1',2',1"]terphenyl-4'-yl)-amine, 14.4 g of 2-(4-bromophenyl)benzoxazole, 4.4 g of sodium t-butoxide, and 60 ml of toluene, and was aerated with nitrogen gas for 30 minutes under ultrasonic irradiation. Then, 0.1 g of palladium acetate and 0.4 ml of a 50% (w/v) toluene solution of tri-(t-butyl)phosphine were added, and the mixture was stirred overnight under reflux with heating. The mixture was left to cool; then, methanol was added thereto, and the precipitated solid was collected, to obtain a crude product. The crude product was subjected to crystallization purification with a toluene/acetone mixed solvent, and the precipitated solid was collected, to obtain 11.0 g of a yellow powder of bis-{4-(benzoxazol-2-yl)phenyl}-([1,1',2',1"]terphenyl-4'-yl)-amine (2-44) (yield: 76.4%).

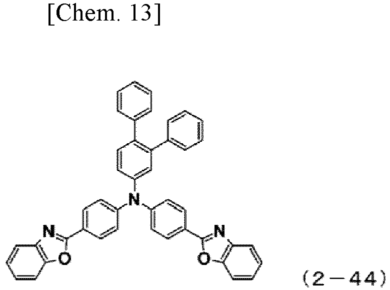

The structure of the obtained yellow powder was identified by NMR.

The following 29 hydrogen signals were detected with ¹H-NMR (DMSO-d₆).

δ (ppm) = 8.19 (4H), 7.78 (4H), 7.50 (1H), 7.45-7.34 (8H), 7.30 (1H), 7.28-7.18 (7H), 7.15 (2H).

### Example 4:

### Synthesis of bis-{4-(benzothiazol-2-yl)phenyl}-{4-(naphthalen-2-yl)phenyl}amine (2-52):

A reaction vessel was charged with 4.7 g of 4-(naphthalen-2-yl)phenyl-amine, 13.7 g of 2-(4-bromophenyl)benzothiazole, 6.2 g of sodium t-butoxide, and 140 ml of toluene, and was aerated with nitrogen gas for 30 minutes under ultrasonic irradiation. Then, 0.6 g of tris(dibenzylideneacetone)dipalladium(0) and 0.6 ml of a 50% (w/v) toluene solution of tri-(t-butyl)phosphine were added, and the mixture was stirred for 3.5 hours under reflux with heating. The mixture was left to cool to 80°C; then, cerite was added thereto and the mixture was filtrated. The filtrate was concentrated, to obtain a crude product. The crude product was dissolved in toluene under heating. Then, activated carbon and silica gel were added at 80°C, and the mixture was filtrated and the filtrate was concentrated. The concentrate was dispersed and washed under reflux with acetone, to obtain 9.3 g of a yellow powder of bis-{4-(benzothiazol-2-yl)phenyl}-{4-(naphthalen-2-yl)phenyl}amine (2-52) (yield: 68%).

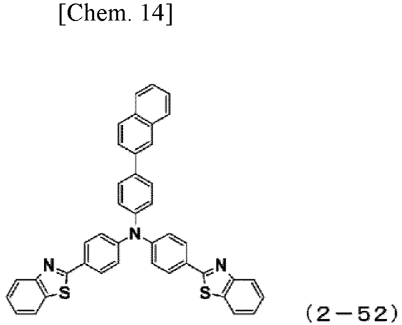

The structure of the obtained yellow powder was identified by NMR.

The following 27 hydrogen signals were detected with ¹H-NMR (DMSO-d₆).

δ (ppm) = 8.27 (1H), 7.88-8.15 (14H), 7.29-7.56 (12H).

### Example 5:

### Synthesis of bis-{4-(benzothiazol-2-yl)phenyl}-{4-(phenanthren-9-yl)phenyl}amine (2-57):

A reaction vessel was charged with 6.0 g of 4-(phenanthren-9-yl)phenyl-amine, 14.2 g of 2-(4-bromophenyl)benzothiazole, 6.4 g of sodium t-butoxide, and 140 ml of toluene, and was aerated with nitrogen gas for 30 minutes under ultrasonic irradiation.

Then, 0.6 g of tris(dibenzylideneacetone)dipalladium(0) and 0.6 ml of a 50% (w/v) toluene solution of tri-(t-butyl)phosphine were added, and the mixture was stirred for 3 hours under reflux with heating. The mixture was left to cool to 80°C; then, cerite was added thereto and the mixture was filtrated. The filtrate was concentrated, to obtain a crude product. The crude product was dissolved in monochlorobenzene under heating. Then, activated carbon and silica gel were added at 80°C, and the mixture was filtrated and the filtrate was concentrated. Acetone was added to the concentrate, and the precipitated solid was filtrated. The obtained solid was recrystallized with toluene, to obtain 10.4 g of a pale yellow powder of bis-{4-(benzothiazol-2-yl)phenyl}-{4-(phenanthren-9-yl)phenyl}amine (2-57) (yield: 68.0%).

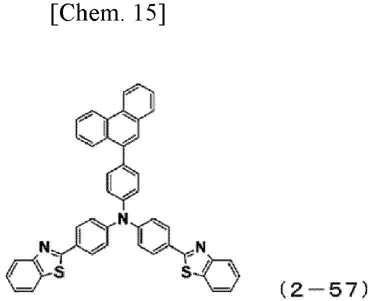

The structure of the obtained pale yellow powder was identified by NMR.

The following 29 hydrogen signals were detected with ¹H-NMR (DMSO-d₆).

δ (ppm) = 8.89-8.99 (2H), 8.00-8.16 (10H), 7.88 (1H), 7.63-7.80 (6H), 7.55 (2H), 7.35-7.48 (8H).

### Example 6:

### Synthesis of bis-{4-(benzothiazol-2-yl)phenyl}-([1,1',2',1"]terphenyl-4'-yl)-amine (2-62):

A reaction vessel was charged with 5.0 g of ([1,1',2',1"]terphenyl-4'-yl)-amine, 13.0 g of 2-(4-bromophenyl)benzothiazole, 59 g of sodium t-butoxide, and 130 ml of toluene, and was aerated with nitrogen gas for 30 minutes under ultrasonic irradiation. Then, 0.6 g of tris(dibenzylideneacetone)dipalladium(0) and 0.6 ml of a 50% (w/v) toluene solution of tri-(t-butyl)phosphine were added, and the mixture was stirred for 18 hours under reflux with heating. The mixture was left to cool to 80°C; then, cerite was added thereto and the mixture was filtrated. The filtrate was concentrated, to obtain a crude product. The crude product was dissolved in toluene under heating. Then, activated carbon and silica gel were added at 80°C, and the mixture was filtrated and the filtrate was concentrated. Acetone was added to the concentrate, and the precipitated solid was filtrated. The obtained solid was recrystallized with toluene, to obtain 7.7 g of a pale yellowish-white powder of bis-{4-(benzothiazol-2-yl)phenyl}-([1,1',2',1"]terphenyl-4'-yl)-amine (2-62) (yield: 57%).

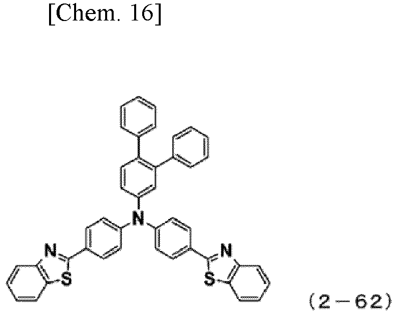

The structure of the obtained pale yellowish-white powder was identified by NMR.

The following 29 hydrogen signals were detected with ¹H-NMR (DMSO-d₆).

δ (ppm) = 8.02-8.15 (8H), 7.09-7.57 (21H).

### Example 7:

### Synthesis of N,N' -bis { 4-(benzoxazol-2-yl)phenyl} -N,N' -diphenyl-4,4' -diamino-1,1' -biphenyl (3-31):

A nitrogen-purged reaction vessel was charged with 13.0 g of 2-(4-bromophenyl)-benzoxazole, 7.6 g of N,N'-diphenylbenzidine, 4.6 g of sodium tert-butoxide, and 160 ml of toluene, and was aerated with nitrogen gas for 30 minutes under ultrasonic irradiation. Then, 0.2 g of palladium acetate and 0.5 g of a 50% (v/v) toluene solution of tert-butylphosphine were added, and the mixture was heated to reflux for 5 hours while being stirred. The mixture was cooled to room temperature and then subjected to filtration to collect the precipitate. Then, purification was performed by column chromatography (adsorbent: NH silica gel; eluent: toluene/n-hexane), followed by dispersion and washing with n-hexane, to obtain 8.8 g of a pale yellow powder of N,N' -bis{4-(benzoxazol-2-yl)phenyl}-N,N'-diphenyl-4,4' -diamino-1,1' -biphenyl (3-31) (yield: 54%).

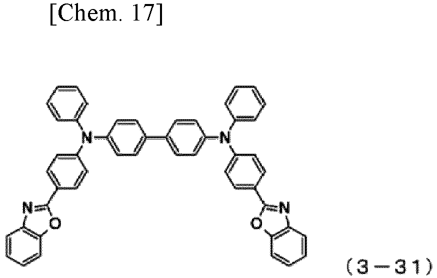

The structure of the obtained pale yellow powder was identified by NMR.

The following 34 hydrogen signals were detected with ¹H-NMR (CDCl₃).

δ (ppm) = 8.12 (4H), 7.80-7.72 (2H), 7.60-7.53 (5H), 7.41-7.14 (23H).

### Example 8:

### Synthesis of N,N'-bis{4-(benzothiazol-2-yl)phenyl}-N,N'-diphenyl-4,4'-diamino-1,1'-biphenyl (3-32):

A nitrogen-purged reaction vessel was charged with 11.0 g of 2-(4-bromophenyl)-benzothiazole, 6.7 g of N,N'-diphenylbenzidine, 3.9 g of sodium tert-butoxide, and 150 ml of toluene, and was aerated with nitrogen gas for 30 minutes under ultrasonic irradiation. Then, 0.2 g of palladium acetate and 0.5 g of a 50% (v/v) toluene solution of tert-butylphosphine were added, and the mixture was heated to reflux for 5 hours while being stirred. The mixture was cooled to room temperature and then subjected to filtration to collect the precipitate. Then, purification was performed by column chromatography (adsorbent: NH silica gel; eluent: toluene/n-hexane), followed by dispersion and washing with n-hexane, to obtain 9.3 g of a pale yellow powder of N,N'-bis{4-(benzothiazol-2-yl)phenyl}-N,N'-diphenyl-4,4'-diamino-1,1'-biphenyl (3-32) (yield: 62%).

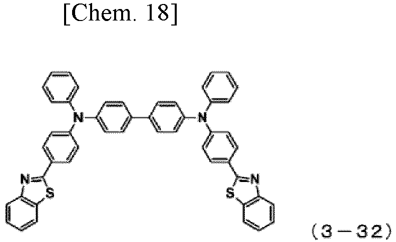

The structure of the obtained pale yellow powder was identified by NMR.

The following 34 hydrogen signals were detected with ¹H-NMR (CDCl₃).

δ (ppm) = 8.10-7.88 (8H), 7.60-7.13 (26H).

### Example 9:

### Synthesis of N,N' -bis { 4-(benzoxazol-2-yl)phenyl} -N,N'-diphenyl-4,4"-diamino-1,1':4',1"-terphenyl (3-56):

A nitrogen-purged reaction vessel was charged with 13.4 g of {4-(benzoxazol-2-yl)phenyl}phenylamine, 10.8 g of 4,4"-1,1' :4',1"-terphenyl, 5.0 g of sodium tert-butoxide, and 150 ml of toluene, and was aerated with nitrogen for 30 minutes under ultrasonic irradiation. Then, 0.2 g of palladium acetate and 0.5 g of a 50% (v/v) toluene solution of tert-butylphosphine were added, and the mixture was heated to reflux for 3 hours while being stirred. The mixture was cooled to room temperature and then subjected to filtration to collect the precipitate. Then, crystallization purification using a 1,2-dichlorobenzene/methanol mixed solvent was repeated, to obtain 8.4 g of a yellow powder of N,N'-bis{4-(benzoxazol-2-yl)phenyl}-N,N'-diphenyl-4,4"-diamino-1,1':4',1"-terphenyl (3-56) (yield: 47%).

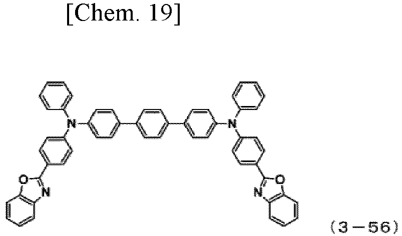

The structure of the obtained yellow powder was identified by NMR.

The following 38 hydrogen signals were detected with ¹H-NMR (CDCl₃).

δ (ppm) = 8.13 (4H), 7.80-7.55 (11H), 7.50-7.16 (23H).

### Example 10:

### Synthesis of N,N'-bis{4-(benzothiazol-2-yl)phenyl}-N,N'-diphenyl-4,4"-diamino-l,1':4',1"-terpheny 1 (3-57):

A nitrogen-purged reaction vessel was charged with 9.3 g of N-{ 4-(benzothiazol-2-yl)phenyl }phenylamine, 7.1 g of 4,4"-diiodo-1,1':4',1"-terphenyl, 4.6 g of sodium tert-butoxide, and 140 ml of toluene, and was aerated with nitrogen for 30 minutes under ultrasonic irradiation. Then, 0.2 g of palladium acetate and 0.5 g of a 50% (v/v) toluene solution of tert-butylphosphine were added, and the mixture was heated to reflux for 3 hours while being stirred. The mixture was cooled to room temperature and then subjected to filtration to collect the precipitate. Then, crystallization purification using a 1,2-dichlorobenzene/methanol mixed solvent was repeated, to obtain 7.0 g of a green powder of N,N'-bis{4-(benzothiazol-2-yl)phenyl}-N,N'-diphenyl-4,4"-diamino-1,1':4',1"-terpheny 1 (3-57) (yield: 58%).

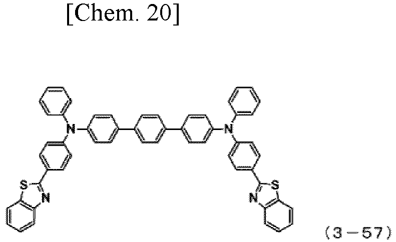

The structure of the obtained yellow powder was identified by NMR.

The following 38 hydrogen signals were detected with ¹H-NMR (CDCl₃).

δ (ppm) = 8.13 (4H), 7.80-7.55 (11H), 7.50-7.16 (23H).

### Example 11:

The melting point and the glass transition point were measured for each of the arylamine compounds obtained in the aforementioned Examples 1 to 10 by using a high-sensitivity differential scanning calorimeter (DSC3100SA from Bruker AXS). The results are shown in Table 1.

**[Table 1]**

| Compound | Melting point | Glass transition point |
|---|---|---|
| Compound (2-34) of Example 1 | 249°C | 111°C |
| Compound (2-39) of Example 2 | Not observed | 133°C |
| Compound (2-44) of Example 3 | Not observed | 120°C |
| Compound (2-52) of Example 4 | Not observed | 112°C |
| Compound (2-57) of Example 5 | Not observed | 135°C |
| Compound (2-62) of Example 6 | Not observed | 120°C |
| Compound (3-31) of Example 7 | 261°C | 128°C |
| Compound (3-32) of Example 8 | 247°C | 127°C |
| Compound (3-56) of Example 9 | Not observed | 137°C |
| Compound (3-57) of Example 10 | 264°C | 137°C |

The results show that the arylamine compounds obtained in Examples 1 to 10 each have a glass transition point of 100°C or higher, and are thus stable in a thin-film state.

### Example 12:

A vapor deposition film having a thickness of 80 nm was formed on a silicon substrate by using each of the arylamine compounds obtained in the aforementioned Examples 1 to 10, and the refractive index n and extinction coefficient k within a wavelength range of 400 nm to 650 nm were measured using a spectrometer (F 10-RT-UV from Filmetrics). For comparison, the refractive index n and extinction coefficient k within a wavelength range of 400 nm to 650 nm were measured also for compounds (CPL-1) and (CPL-2) having the following structural formulas (see, for example, Patent Literature 6). The measurement results are collectively shown in Table 2.

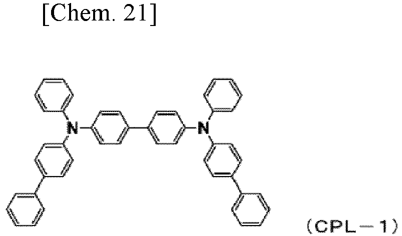

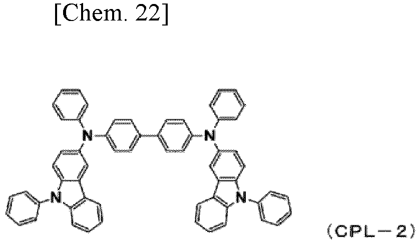

**[Table 2]**

| Compound | | 400 nm | 410 nm | 450 nm | 500 nm | 550 nm | 600 nm | 650 nm |
|---|---|---|---|---|---|---|---|---|
| 2-34 | n: Refractive index | 2.50 | 2.56 | 2.30 | 2.09 | 2.01 | 1.96 | 1.94 |
| | k: Extinction coefficient | 0.75 | 0.51 | 0.06 | 0.00 | 0.00 | 0.00 | 0.00 |
| 2-39 | n: Refractive index | 2.49 | 2.60 | 2.28 | 2.09 | 2.02 | 1.98 | 1.96 |
| | k: Extinction coefficient | 0.72 | 0.48 | 0.01 | 0.00 | 0.00 | 0.00 | 0.00 |
| 2-44 | n: Refractive index | 2.36 | 2.47 | 2.18 | 2.01 | 1.95 | 1.92 | 1.90 |
| | k: Extinction coefficient | 0.67 | 0.46 | 0.00 | 0.00 | 0.00 | 0.00 | 0.00 |
| 2-52 | n: Refractive index | 2.21 | 2.39 | 2.36 | 2.12 | 2.01 | 1.96 | 1.93 |
| | k: Extinction coefficient | 0.89 | 0.75 | 0.15 | 0.01 | 0.00 | 0.00 | 0.00 |
| 2-57 | n: Refractive index | 2.25 | 2.42 | 2.39 | 2.13 | 2.05 | 2.00 | 1.97 |
| | k: Extinction coefficient | 0.79 | 0.69 | 0.09 | 0.00 | 0.00 | 0.00 | 0.00 |
| 2-62 | n: Refractive index | 2.18 | 2.35 | 2.29 | 2.07 | 1.98 | 1.94 | 1.92 |
| | k: Extinction coefficient | 0.77 | 0.64 | 0.09 | 0.00 | 0.00 | 0.00 | 0.00 |
| 3-31 | n: Refractive index | 2.61 | 2.69 | 2.27 | 2.06 | 1.98 | 1.94 | 1.91 |
| | k: Extinction coefficient | 0.81 | 0.50 | 0.01 | 0.00 | 0.00 | 0.00 | 0.00 |
| 3-32 | n: Refractive index | 2.23 | 2.43 | 2.31 | 2.08 | 2.00 | 1.95 | 1.93 |
| | k: Extinction coefficient | 0.84 | 0.67 | 0.04 | 0.00 | 0.00 | 0.00 | 0.00 |
| 3-56 | n: Refractive index | 2.55 | 2.62 | 2.26 | 2.05 | 1.98 | 1.94 | 1.91 |
| | k: Extinction coefficient | 0.78 | 0.49 | 0.02 | 0.00 | 0.00 | 0.00 | 0.00 |
| 3-57 | n: Refractive index | 226 | 2.45 | 2.28 | 2.04 | 1.97 | 1.93 | 1.90 |
| | k: Extinction coefficient | 0.83 | 0.63 | 0.05 | 0.00 | 0.00 | 0.00 | 0.00 |
| CPL-1 | n: Refractive index | 2.21 | 2.15 | 1.98 | 1.89 | 1.85 | 1.82 | 1.81 |
| | k: Extinction coefficient | 0.17 | 0.10 | 0.00 | 0.00 | 0.00 | 0.00 | 0.00 |
| CPL-2 | n: Refractive index | 2.28 | 2.22 | 2.02 | 1.94 | 1.90 | 1.87 | 1.84 |
| | k: Extinction coefficient | 0.13 | 0.03 | 0.00 | 0.00 | 0.00 | 0.00 | 0.00 |

As shown in Table 1, compared to the compounds (CPL-1) and (CPL-2), the arylamine compounds according to the present invention having a group represented by the formula (1) each have a high refractive index in the blue light emission wavelength range (450 nm), wherein the compounds of the present invention have refractive indices of 2.18 to 2.39, whereas the compounds (CPL-1) and (CPL-2) have refractive indices of 1.98 and 2.02, respectively. Also, in the green light emission wavelength range (550 nm), the compounds of the present invention have high refractive indices ranging from 1.95 to 2.05, whereas the compounds (CPL-1) and (CPL-2) have refractive indices of 1.85 and 1.90, respectively. Also, in the red light emission wavelength range (650 nm), the compounds of the present invention have high refractive indices ranging from 1.90 to 1.97, whereas the compounds (CPL-1) and (CPL-2) have refractive indices of 1.84 and 1.81, respectively.

As described above, the compounds according to the present invention have higher refractive indices than the compounds (CPL-1) and (CPL-2) in the blue, green and red light emission wavelength ranges, and can therefore be expected to improve light extraction efficiency in organic EL devices.

Further, as regards the extinction coefficient within the wavelength range of 400 nm to 410 nm, the compounds of the present invention have large values ranging from 0.46 to 0.89, whereas the compounds (CPL-1) and (CPL-2) have values below 0.2. This shows that the present compounds favorably absorb sunlight within the wavelength range of 400 nm to 410 nm and therefore prevent impact on materials inside devices.

### Example 13:

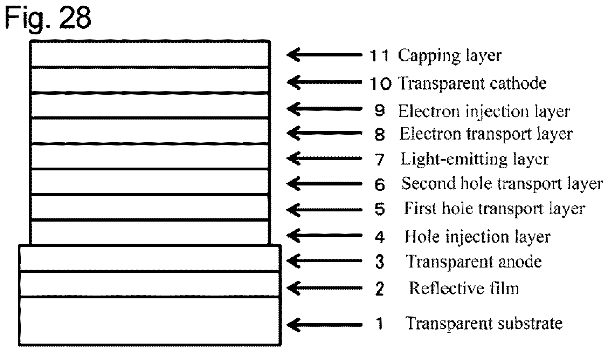

As illustrated in Fig. 28, an organic EL device was prepared by vapor-depositing a hole injection layer 4, a first hole transport layer 5, a second hole transport layer 6, a light-emitting layer 7, an electron transport layer 8, an electron injection layer 9, a cathode 10, and a capping layer 11 in this order onto a glass substrate 1 having formed thereon a reflective film 2 and an ITO electrode as a metal anode 3 in advance.

More specifically, a glass substrate 1 having formed thereon a reflective film 2 and ITO as a transparent anode 3 was subjected to ultrasonic cleaning in isopropyl alcohol for 20 minutes, and then dried for 10 minutes on a hot plate heated to 200°C. Then, after UV ozone treatment for 15 minutes, the glass substrate having the reflective film and ITO was mounted to a vacuum vapor deposition apparatus, in which the pressure was reduced to 0.001 Pa or lower. Next, a hole injection layer 4 was formed so as to cover the transparent anode 3 by performing binary vapor deposition, onto the transparent anode 3, of a compound (Acceptor-1) and a compound (HTM-1) respectively having the following structural formulas. The vapor deposition was performed at a rate at which the vapor deposition rate ratio between Acceptor-1 and HTM-1 was 3:97. The hole injection layer 4 was formed having a film thickness of 10 nm.

On this hole injection layer 4, HTM-1 was vapor-deposited to form a first hole transport layer 5 having a film thickness of 70 nm.

On this first hole transport layer 5, an arylamine compound (5-48) having the following structural formula was vapor-deposited to form a second hole transport layer 6 having a film thickness of 10 nm.

On this second hole transport layer 6, a light-emitting layer 7 was formed by simultaneously employing, as a host, a first host compound (A-43) having the following structural formula and a second host compound (B-25) having the following structural formula while doping, as a dopant, a metal complex (4-35) having the following structural formula at 5 wt%, and performing vacuum vapor deposition of the compounds, to form the light-emitting layer 7 having a film thickness of 40 nm. Herein, the first host compound (A-43) and the second host compound (B-25) were employed at a 1: 1 ratio.

On this light-emitting layer 7, an electron transport layer 8 was formed by performing binary vapor deposition of a compound (ETM-1) having the following structural formula and a compound (ETM-2) having the following structural formula. The vapor deposition was performed at a rate at which the vapor deposition rate ratio between ETM-1 and ETM-2 was 50:50. The electron transport layer 8 was formed having a film thickness of 30 nm.

On this electron transport layer 8, lithium fluoride was vapor-deposited to form an electron injection layer 9 having a film thickness of 1 nm.

On this electron injection layer 9, a magnesium silver alloy layer was formed as a transparent cathode 10 having a film thickness of 12 nm.

Finally, the compound (2-34) obtained in Example 1 was formed as a capping layer 11 having a film thickness of 60 nm. Properties of the produced organic EL device were measured in the atmosphere at atmospheric temperature.

Measurement results of light emission properties when a direct-current voltage was applied to the produced organic EL device are collectively shown in Table 3.

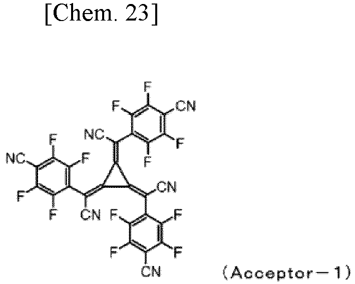

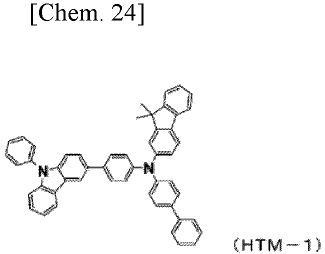

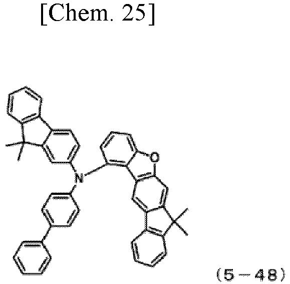

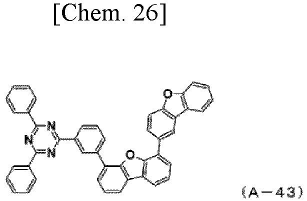

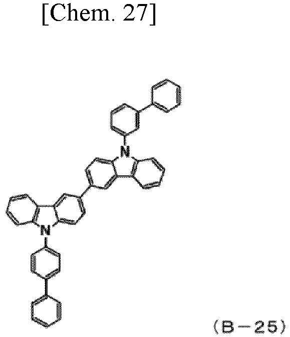

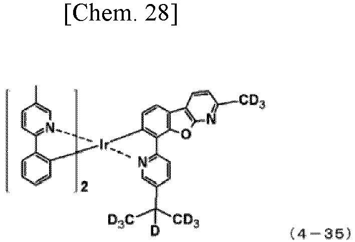

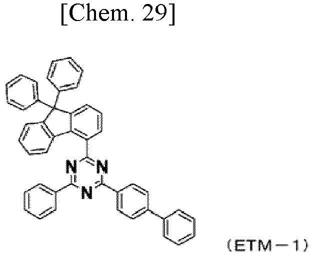

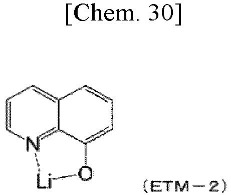

### Examples 14 to 22:

Organic EL devices were produced in the same manner as above, except that the respective compounds obtained in Examples 2 to 10 were used instead of the compound (2-34) obtained in Example 1 used as the material for the capping layer 11 in Example 13. Properties of the produced organic EL devices were measured in the atmosphere at atmospheric temperature. Measurement results of light emission properties when a direct-current voltage was applied to each of the produced organic EL devices are collectively shown in Table 3.

Device life was measured using the organic EL devices produced in Examples 13 to 22 and Comparative Examples 1 and 2. The results are collectively shown in Table 3. Device life was measured as follows. Constant current driving was performed, with the light emission luminance at the start of light emission (i.e., initial luminance) being 10000 cd/m², and the time (hours) it took for the light emission luminance to attenuate to 9500 cd/m² (95% attenuation: amounting to 95% when the initial luminance is considered 100%) was measured.

**[Table 3]**

| | Compound | Voltage [V] (@10 mA/cm²) | Luminance [cd/m²] (@10 mA/cm²) | Luminous efficiency [cd/A] (@10 mA/cm²) | Power efficiency [lmlW] (@10 mA/cm²) | Device life, 95% attenuation |
|---|---|---|---|---|---|---|
| Example 13 | 2-34 | 4.25 | 12226 | 122.26 | 90.38 | 415 hours |
| Example 14 | 2-39 | 4.24 | 12552 | 125.52 | 93.01 | 432 hours |
| Example 15 | 2-44 | 4.24 | 11834 | 11834 | 87.69 | 402 hours |
| Example 16 | 2-52 | 4.23 | 12055 | 120.55 | 89.54 | 410 hours |
| Example 17 | 2-57 | 4.23 | 12484 | 124.84 | 92.73 | 415 hours |
| Example 18 | 2-62 | 4.23 | 12330 | 12330 | 91.58 | 414 hours |
| Example 19 | 3-31 | 4.24 | 12443 | 124.43 | 92.20 | 426 hours |
| Example 20 | 3-32 | 4.23 | 12061 | 120.61 | 89.59 | 402 hours |
| Example 21 | 3-56 | 4.25 | 12001 | 120.01 | 88.72 | 408 hours |
| Example 22 | 3-57 | 4.24 | 11937 | 119.37 | 88.45 | 400 hours |
| Comparative Example 1 | CPL-1 | 4.24 | 10210 | 102.10 | 75.66 | 304 hours |
| Comparative Example 2 | CPL-2 | 4.24 | 11234 | 112.34 | 83.25 | 362 hours |

As shown in Table 3, the drive voltage at a current density of 10 mA/cm² was substantially the same for the devices of Comparative Examples 1 and 2 and the devices of Examples 13 to 22. However, the devices of Examples 13 to 22 had improved luminance, luminous efficiency, power efficiency and lifetime, compared to the devices of Comparative Examples 1 and 2. This shows that light extraction efficiency can be significantly improved by containing, in the capping layer, materials according to the present invention that have a high refractive index and are suitably usable in organic EL devices.

### Industrial Applicability

As described above, the arylamine compound having a group represented by the aforementioned formula (1), which is suitably usable in the organic EL device of the present invention, has a high refractive index, and can thus significantly improve light extraction efficiency. Also, the present arylamine compound is stable in a thin-film state, and is thus an excellent compound for use in an organic EL device. By producing an organic EL device by using the present compound and a metal complex as a phosphorescent dopant, high efficiency can be achieved. Further, by using the present compound in a capping layer of an organic EL device, sunlight within a specific wavelength range can be absorbed, thereby preventing impact on materials inside the device and thus improving durability and light resistance. Further, by using the present compound which has no absorption in the blue, green and red wavelength ranges, it is possible to display clear and bright images with excellent color purity. The devices are thus applicable to, for example, household electrical appliances and illumination.

## Claims

1. An organic electroluminescence device comprising at least an anode (3), a first hole transport layer (5), a second hole transport layer (6), a light-emitting layer (7), an electron transport layer (8), a cathode (10), and a capping layer (11) in this order, wherein:
the capping layer (11) contains an arylamine compound having a group represented by the following formula (1); and
the light-emitting layer (7) contains a host and a phosphorescent dopant: 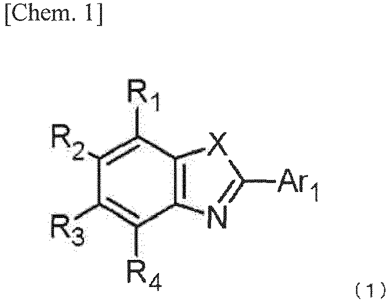
(in the formula, R₁ to R₄ each independently represent a hydrogen atom, a deuterium atom, a fluorine atom, a chlorine atom, a cyano group, a nitro group, a silyl group, a linear or branched alkyl group having 1 to 6 carbon atoms and optionally having a substituent, a cycloalkyl group having 5 to 10 carbon atoms and optionally having a substituent, a linear or branched alkenyl group having 2 to 6 carbon atoms and optionally having a substituent, a linear or branched alkyloxy group having 1 to 6 carbon atoms and optionally having a substituent, a cycloalkyloxy group having 5 to 10 carbon atoms and optionally having a substituent, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group;
Ar₁ represents a substituted or unsubstituted aromatic hydrocarbon group, a substituted or unsubstituted aromatic heterocyclic group, or a substituted or unsubstituted fused polycyclic aromatic group;
one of R₁ to R₄ and An is a linking group as a bonding site;
X represents an oxygen atom or a sulfur atom; and
R₁ to R₄ may be bonded to each other via a single bond, a substituted or unsubstituted methylene group, a substituted or unsubstituted amino group, an oxygen atom, or a sulfur atom, to form a ring);
**characterized in that** the host contains a first host compound represented by the following formula (Host-A) and a second host compound represented by the following formula (Host-B):
Host A: 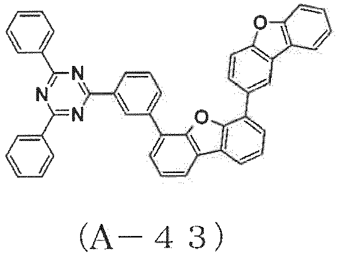
Host B: 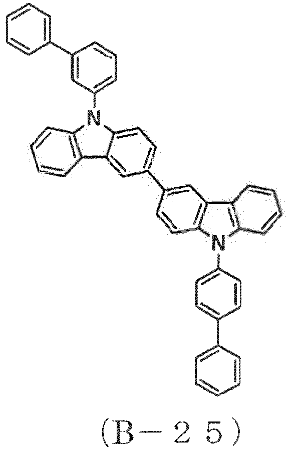
and that the phosphorescent dopant is a metal complex represented by the following formula:

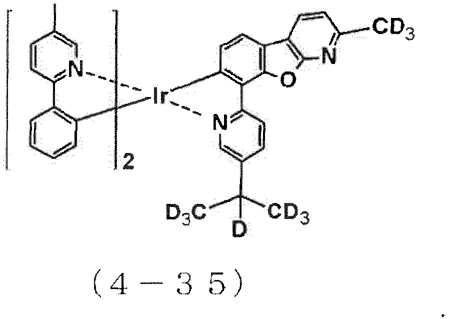

2. The organic electroluminescence device according to claim 1, wherein the arylamine compound having a group represented by the formula (1) is an arylamine compound represented by the following formula (2) or formula (3): 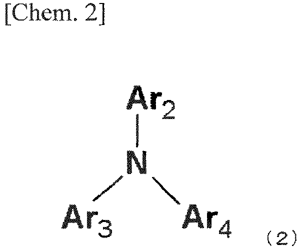
(in the formula, Ar₂, Ar₃, and Ar₄ each independently represent a substituted or unsubstituted aromatic hydrocarbon group, a substituted or unsubstituted aromatic heterocyclic group, a substituted or unsubstituted fused polycyclic aromatic group, or a group represented by the formula (1), wherein at least one of Ar₂, Ar₃, and Ar₄ is a group represented by the formula (1)); 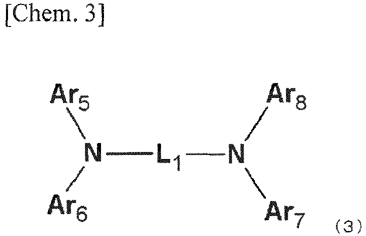
(in the formula, Ar₅, Ar₆, Ar₇, and Ar₈ each independently represent a substituted or unsubstituted aromatic hydrocarbon group, a substituted or unsubstituted aromatic heterocyclic group, a substituted or unsubstituted fused polycyclic aromatic group, or a group represented by the formula (1), wherein at least one of Ar₅, Ar₆, Ar₇, and Ar₈ is a group represented by the formula (1); and
L₁ represents a substituted or unsubstituted phenylene group, a substituted or unsubstituted biphenylene group, or a substituted or unsubstituted terphenylene group).

3. The organic electroluminescence device according to claim 2, wherein the arylamine compound having a group represented by the formula (1) is an arylamine compound represented by the formula (3).

4. The organic electroluminescence device according to any one of claims 1 to 3, wherein the second hole transport layer (6) contains an arylamine compound represented by the following formula (5): 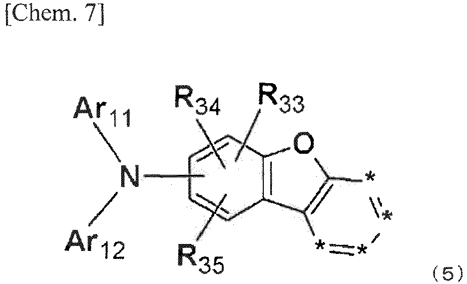
(in the formula, adjacent two individuals * bond with two individuals * in the following formula (6) to form a ring, and the remaining two individuals * represent CRb and CRc;
R₃₃ to R₃₅, Rb, and Rc each independently represent a hydrogen atom, a deuterium atom, a fluorine atom, a chlorine atom, a cyano group, a nitro group, a substituted or unsubstituted alkyl group having 1 to 15 carbon atoms, or a substituted or unsubstituted aryl group having 6 to 12 carbon atoms; and
Ar₁₁ and Ar₁₂ each independently represent a substituted or unsubstituted aryl group having 6 to 30 carbon atoms, or a substituted or unsubstituted heteroaryl group having 5 to 30 carbon atoms); 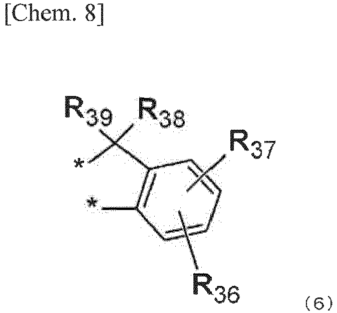
(in the formula, R₃₆ to R₃₉, Rb, and Rc each independently represent a hydrogen atom, a deuterium atom, a fluorine atom, a chlorine atom, a cyano group, a nitro group, a substituted or unsubstituted alkyl group having 1 to 15 carbon atoms, or a substituted or unsubstituted aryl group having 6 to 12 carbon atoms).

5. The organic electroluminescence device according to any one of claims 1 to 4, wherein the capping layer (11) has a thickness within a range of 30 nm to 120 nm.

6. The organic electroluminescence device according to any one of claims 1 to 5, wherein the capping layer (11) has a refractive index of 1.85 or greater within a wavelength range of 450 nm to 650 nm.

## Patentansprüche

1. Organische Elektrolumineszenzvorrichtung, umfassend mindestens eine Anode (3), eine erste Lochtransportschicht (5), eine zweite Lochtransportschicht (6), eine lichtemittierende Schicht (7), eine Elektronentransportschicht (8), eine Kathode (10) und eine Deckschicht (11) in dieser Reihenfolge, wobei:
die Deckschicht (11) eine Arylaminverbindung enthält, die eine Gruppe aufweist, die durch die folgende Formel (1) dargestellt ist; und
die lichtemittierende Schicht (7) einen Host und einen phosphoreszierenden Dotierstoff enthält: 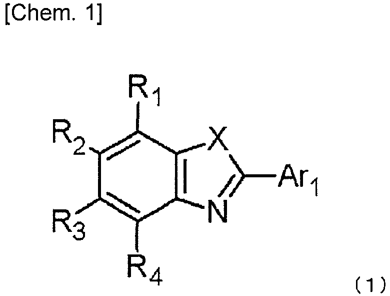
(in der Formel repräsentieren R₁ bis R₄ jeweils unabhängig voneinander ein Wasserstoffatom, ein Deuteriumatom, ein Fluoratom, ein Chloratom, eine Cyanogruppe, eine Nitrogruppe, eine Silylgruppe, eine lineare oder verzweigte Alkylgruppe mit 1 bis 6 Kohlenstoffatomen und optional mit einem Substituenten, eine Cycloalkylgruppe mit 5 bis 10 Kohlenstoffatomen und optional mit einem Substituenten, eine lineare oder verzweigte Alkenylgruppe mit 2 bis 6 Kohlenstoffatomen und optional mit einem Substituenten, eine lineare oder verzweigte Alkyloxygruppe mit 1 bis 6 Kohlenstoffatomen und optional mit einem Substituenten, eine Cycloalkyloxygruppe mit 5 bis 10 Kohlenstoffatomen und optional mit einem Substituenten, eine substituierte oder unsubstituierte aromatische Kohlenwasserstoffgruppe, eine substituierte oder unsubstituierte aromatische heterocyclische Gruppe, eine substituierte oder unsubstituierte kondensierte polycyclische aromatische Gruppe oder eine substituierte oder unsubstituierte Aryloxygruppe;
Ar₁ repräsentiert eine substituierte oder unsubstituierte aromatische Kohlenwasserstoffgruppe, eine substituierte oder unsubstituierte aromatische heterozyklische Gruppe oder eine substituierte oder unsubstituierte kondensierte polyzyklische aromatische Gruppe;
eines von R₁ bis R₄ und Ar₁ ist eine verbindende Gruppe als Bindungsstelle;
X repräsentiert ein Sauerstoffatom oder ein Schwefelatom; und
R₁ bis R₄ können über eine Einfachbindung, eine substituierte oder unsubstituierte Methylengruppe, eine substituierte oder unsubstituierte Aminogruppe, ein Sauerstoffatom oder ein Schwefelatom miteinander verbunden sein, um einen Ring zu bilden);
**dadurch gekennzeichnet, dass** der Host eine erste Hostverbindung, die durch die folgende Formel (Host-A) dargestellt wird, und eine zweite Hostverbindung, die durch die folgende Formel (Host-B) dargestellt wird, enthält:
Host-A: 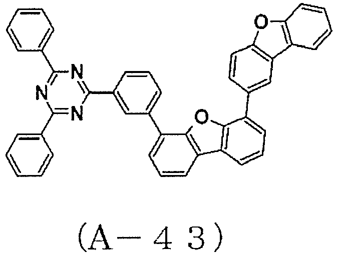
Host-B 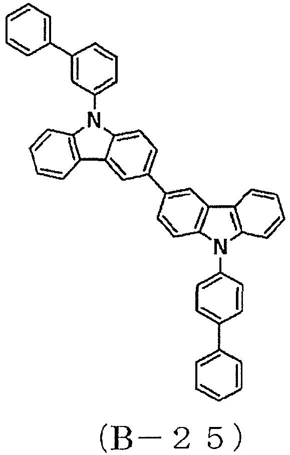
und dass der phosphoreszierende Dotierstoff ein Metallkomplex ist, der durch die folgende Formel dargestellt wird:

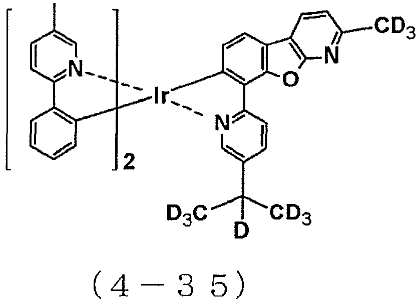

2. Organische Elektrolumineszenzvorrichtung nach Anspruch 1, wobei die Arylaminverbindung, die eine Gruppe aufweist, die durch die Formel (1) dargestellt wird, eine durch die folgende Formel (2) oder Formel (3) dargestellte Arylaminverbindung ist: 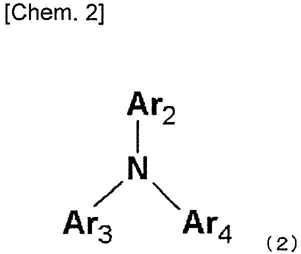
(in der Formel stellen Ar₂, Ar₃ und Ar₄ jeweils unabhängig voneinander eine substituierte oder unsubstituierte aromatische Kohlenwasserstoffgruppe, eine substituierte oder unsubstituierte aromatische heterocyclische Gruppe, eine substituierte oder unsubstituierte kondensierte polycyclische aromatische Gruppe oder eine Gruppe dar, die durch die Formel (1) dargestellt wird, wobei mindestens eines von Ar₂, Ar₃ und Ar₄ eine Gruppe ist, die durch die Formel (1) dargestellt wird); 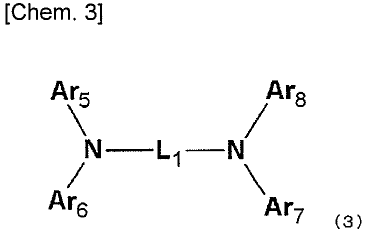
(in der Formel stellen Ar₅, Ar₆, Ar₇ und Ar₈ jeweils unabhängig voneinander eine substituierte oder unsubstituierte aromatische Kohlenwasserstoffgruppe, eine substituierte oder unsubstituierte aromatische heterocyclische Gruppe, eine substituierte oder unsubstituierte kondensierte polycyclische aromatische Gruppe oder eine durch die Formel (1) dargestellte Gruppe dar, wobei mindestens eines von Ar₅, Ar₆, Ar₇ und Ar₈ eine durch die Formel (1) dargestellte Gruppe ist; und
L₁ eine substituierte oder unsubstituierte Phenylengruppe, eine substituierte oder unsubstituierte Biphenylengruppe oder eine substituierte oder unsubstituierte Terphenylengruppe darstellt).

3. Organische Elektrolumineszenzvorrichtung nach Anspruch 2, wobei die Arylaminverbindung, die eine Gruppe aufweist, die durch die Formel (1) dargestellt wird, eine durch die Formel (3) dargestellte Arylaminverbindung ist.

4. Organische Elektrolumineszenzvorrichtung nach einem der Ansprüche 1 bis 3, wobei die zweite Lochtransportschicht (6) eine Arylaminverbindung enthält, die durch die folgende Formel (5) dargestellt wird:) 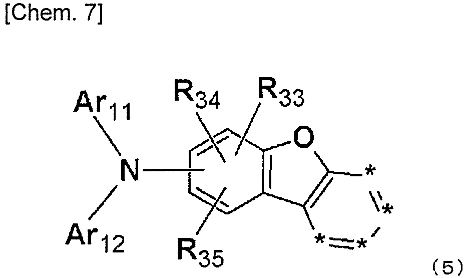
(in der Formel verbinden sich zwei benachbarte Individuen * mit zwei Individuen * in der folgenden Formel (6), um einen Ring zu bilden, und die verbleibenden zwei Individuen * stellen CRb und CRc dar;
R₃₃ bis R₃₅, Rb und Rc stellen jeweils unabhängig voneinander ein Wasserstoffatom, ein Deuteriumatom, ein Fluoratom, ein Chloratom, eine Cyanogruppe, eine Nitrogruppe, eine substituierte oder unsubstituierte Alkylgruppe mit 1 bis 15 Kohlenstoffatomen oder eine substituierte oder unsubstituierte Arylgruppe mit 6 bis 12 Kohlenstoffatomen dar; und
Ar₁₁ und Ar₁₂ stellen jeweils unabhängig voneinander eine substituierte oder unsubstituierte Arylgruppe mit 6 bis 30 Kohlenstoffatomen oder eine substituierte oder unsubstituierte Heteroarylgruppe mit 5 bis 30 Kohlenstoffatomen dar); 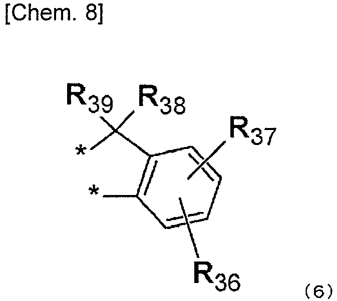
(in der Formel stellen R₃₆ bis R₃₉, Rb und Rc jeweils unabhängig voneinander ein Wasserstoffatom, ein Deuteriumatom, ein Fluoratom, ein Chloratom, eine Cyanogruppe, eine Nitrogruppe, eine substituierte oder unsubstituierte Alkylgruppe mit 1 bis 15 Kohlenstoffatomen oder eine substituierte oder unsubstituierte Arylgruppe mit 6 bis 12 Kohlenstoffatomen dar).

5. Organische Elektrolumineszenzvorrichtung nach einem der Ansprüche 1 bis 4, wobei die Deckschicht (11) eine Dicke in einem Bereich von 30 nm bis 120 nm aufweist.

6. Organische Elektrolumineszenzvorrichtung nach einem der Ansprüche 1 bis 5, wobei die Deckschicht (11) einen Brechungsindex von 1,85 oder größer in einem Wellenlängenbereich von 450 nm bis 650 nm aufweist.

## Revendications

1. Dispositif organique à électroluminescence comprenant au moins une anode (3), une première couche de transport de trous (5), une deuxième couche de transport de trous (6), une couche émettrice de lumière (7), une couche de transport d'électrons (8), une cathode (10) et une couche de recouvrement (11) dans cet ordre, dans lequel:
la couche de recouvrement (11) contient un composé d'arylamine ayant un groupe représenté par la formule suivante (1); et
la couche émettrice de lumière (7) contient un hôte et un dopant phosphorescent: 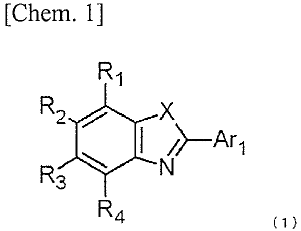
(dans la formule, R₁ à R₄ représentent chacun indépendamment un atome d'hydrogène, un atome de deutérium, un atome de fluor, un atome de chlore, un groupe cyano, un groupe nitro, un groupe silyle, un groupe alkyle linéaire ou ramifié ayant de 1 à 6 atomes de carbone et ayant éventuellement un substituant, un groupe cycloalkyle ayant de 5 à 10 atomes de carbone et ayant éventuellement un substituant, un groupe alcényle linéaire ou ramifié ayant de 2 à 6 atomes de carbone et ayant éventuellement un substituant, un groupe alkyloxy linéaire ou ramifié ayant de 1 à 6 atomes de carbone et ayant éventuellement un substituant, un groupe cycloalkyloxy ayant de 5 à 10 atomes de carbone et ayant éventuellement un substituant, un groupe hydrocarbure aromatique substitué ou non-substitué, un groupe hétérocyclique aromatique substitué ou non substitué, un groupe aromatique polycyclique fusionné substitué ou non-substitué, ou un groupe aryloxy substitué ou non-substitué;
An représente un groupe d'hydrocarbures aromatiques substitué ou non-substitué, un groupe hétérocyclique aromatique substitué ou non-substitué, ou un groupe aromatique polycyclique fusionné substitué ou non-substitué;
l'un des Ri à R₄ et Ar₁ est un groupe de liaison en tant que site de liaison;
X représente un atome d'oxygène ou un atome de soufre; et
Ri à R₄ peuvent être liés l'un à l'autre par une liaison simple, un groupe méthylène substitué ou non-substitué, un groupe amino substitué ou non-substitué, un atome d'oxygène ou un atome de soufre, pour former un anneau);
**caractérisé par le fait que** l'hôte contient un premier composé hôte représenté par la formule suivante (Host-A) et un second composé hôte représenté par la formule suivante (Host-B) :
Host-A : 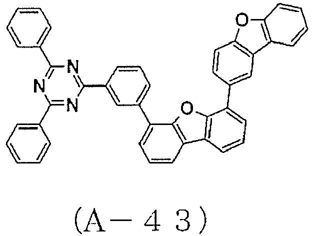
Host-B : 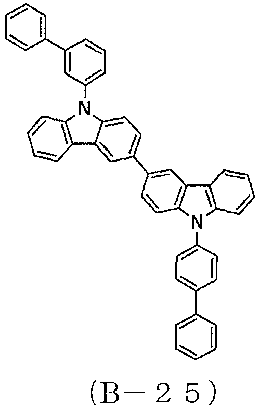
et que le dopant phosphorescent est un complexe métallique représenté par la formule suivante :

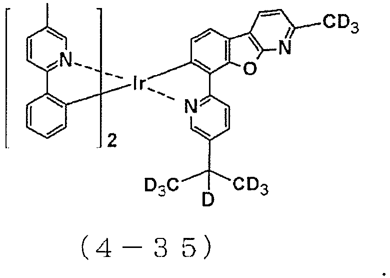

2. Dispositif organique à électroluminescence selon la revendication 1, dans lequel le composé d'arylamine ayant un groupe représenté par la formule (1) est un composé d'arylamine représenté par la formule (2) ou la formule (3) suivante: 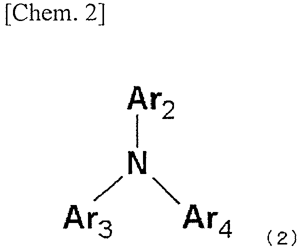
(dans la formule, Ar₂ , Ar₃ , et Ar₄ représentent chacun indépendamment un groupe d'hydrocarbures aromatiques substitué ou non substitué, un groupe hétérocyclique aromatique substitué ou non substitué, un groupe aromatique polycyclique fusionné substitué ou non substitué, ou un groupe représenté par la formule (1), où au moins un de Ar₂ , Ar₃ , et Ar₄ est un groupe représenté par la formule (1)); 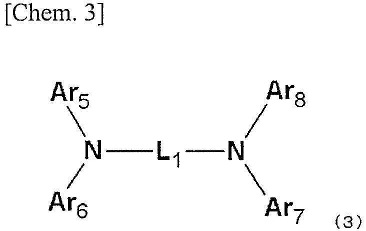
(dans la formule, Ar₅ , Ar₆ , Ar₇ , et Ar₈ représentent chacun indépendamment un groupe d'hydrocarbures aromatiques substitué ou non-substitué, un groupe hétérocyclique aromatique substitué ou non-substitué, un groupe aromatique polycyclique fusionné substitué ou non-substitué, ou un groupe représenté par la formule (1), dans lequel au moins un de Ar₅ , Ar₆ , Ar₇ , et Ar₈ est un groupe représenté par la formule (1); et
L₁ représente un groupe phénylène substitué ou non-substitué, un groupe biphénylène substitué ou non-substitué, ou un groupe terphénylène substitué ou non-substitué).

3. Dispositif organique à électroluminescence selon la revendication 2, dans lequel le composé d'arylamine ayant un groupe représenté par la formule (1) est un composé d'arylamine représenté par la formule (3).

4. Dispositif organique à électroluminescence selon l'une quelconque des revendications 1 à 3, dans lequel la seconde couche de transport de trous (6) contient un composé d'arylamine représenté par la formule suivante (5): 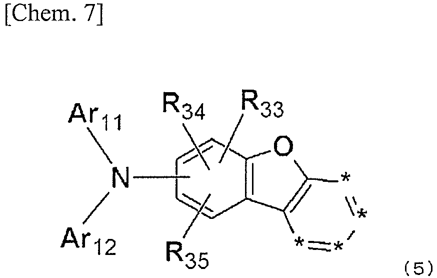
(dans la formule, deux individus adjacents * se lient à deux individus * dans la formule suivante (6) pour former un anneau, et les deux individus restants * représentent CRb et CRc;
R₃₃ à R₃₅, Rb et Rc représentent chacun indépendamment un atome d'hydrogène, un atome de deutérium, un atome de fluor, un atome de chlore, un groupe cyano, un groupe nitro, un groupe alkyle substitué ou non-substitué ayant de 1 à 15 atomes de carbone, ou un groupe aryle substitué ou non-substitué ayant de 6 à 12 atomes de carbone; et
Ar₁₁ et Ar₁₃ représentent chacun indépendamment un groupe aryle substitué ou non-substitué ayant 6 à 30 atomes de carbone, ou un groupe hétéroaryle substitué ou non-substitué ayant 5 à 30 atomes de carbone); 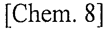 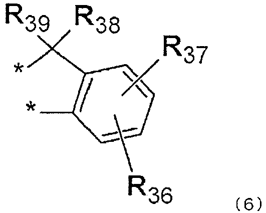
(dans la formule, R₃₆ à R₃₉ , Rb et Rc représentent chacun indépendamment un atome d'hydrogène, un atome de deutérium, un atome de fluor, un atome de chlore, un groupe cyano, un groupe nitro, un groupe alkyle substitué ou non-substitué ayant de 1 à 15 atomes de carbone, ou un groupe aryle substitué ou non-substitué ayant de 6 à 12 atomes de carbone).

5. Dispositif organique à électroluminescence selon l'une des revendications 1 à 4, dans lequel la couche de recouvrement (11) a une épaisseur comprise entre 30 nm et 120 nm.

6. Dispositif organique à électroluminescence selon l'une des revendications 1 à 5, dans lequel la couche de recouvrement (11) a un indice de réfraction de 1,85 ou plus dans une plage de longueurs d'onde de 450 nm à 650 nm.
